(19) Europäisches Patentamt
European Patent Office
Office européen des brevets

(11) EP 3 724 204 B1

(12) **EUROPÄISCHE PATENTSCHRIFT**

(45) Veröffentlichungstag und Bekanntmachung des Hinweises auf die Patenterteilung:
**21.08.2024 Patentblatt 2024/34**

(21) Anmeldenummer: **18814654.2**

(22) Anmeldetag: **12.12.2018**

(51) Internationale Patentklassifikation (IPC):
***C07F 17/02*** (2006.01) ***C07F 5/00*** (2006.01)
***C07F 5/06*** (2006.01) ***C23C 16/00*** (2006.01)
***C30B 25/00*** (2006.01) ***C07C 245/24*** (2006.01)
***C23C 16/18*** (2006.01)

(52) Gemeinsame Patentklassifikation (CPC):
**C07F 5/066; C07C 245/24; C07F 5/00; C07F 5/003; C07F 17/02; C23C 16/18**

(86) Internationale Anmeldenummer:
**PCT/EP2018/084628**

(87) Internationale Veröffentlichungsnummer:
**WO 2019/115646 (20.06.2019 Gazette 2019/25)**

(54) **METALLKOMPLEXE MIT TRIAZENIDOLIGANDEN UND DEREN VERWENDUNGEN ZUR ABSCHEIDUNG VON METALLEN AUS DER GASPHASE**

METAL COMPLEXES WITH TRIAZENIDO LIGANDS AND USES THEREOF FOR THE DEPOSITION OF METALS FROM THE GAS PHASE

COMPLEXES MÉTALLIQUES COMPORTANT DES TRIAZÉNIDOLIGANDES ET LEURS UTILISATIONS PERMETTANT LA SÉPARATION DE MÉTAUX DE LA PHASE GAZEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017 EP 17207806**

(43) Veröffentlichungstag der Anmeldung:
**21.10.2020 Patentblatt 2020/43**

(73) Patentinhaber: **Umicore AG & Co. KG**
**63457 Hanau-Wolfgang (DE)**

(72) Erfinder:
- **SUNDERMEYER, Joerg**
  **35041 Marburg (DE)**
- **HERRITSCH, Susanne**
  **35039 Marburg (DE)**
- **SCHRÖDER, Fabian**
  **38118 Braunschweig (DE)**

(74) Vertreter: **Clauswitz, Kai-Uwe Wolfram**
**Umicore AG & Co. KG**
**Rodenbacher Chaussee 4**
**63457 Hanau-Wolfgang (DE)**

(56) Entgegenhaltungen:
**US-A- 3 386 985**

- **BRINCKMAN F E ET AL: "METAL-NITROGEN BONDING. COVALENT COMPLEXES OF 1,3-DIMETHYLTRIAZENE WITH ELEMENTS OF GROUPS I, II, III, IV, AND V", INORGANIC CHEMISTRY, AMERICAN CHEMICAL SOCIETY, EASTON , US, vol. 4, no. 7, 1 January 1965 (1965-01-01), pages 936 - 942, XP001188971, ISSN: 0020-1669, DOI: 10.1021/IC50029A003**
- **DIMROTH OTTO: "Ueber das Diazoamino-methan (Dimethyl-triazen)", BERICHTE DER DEUTSCHEN CHEMISCHEN GESELLSCHAFT, vol. 39, no. 4, 1 November 1906 (1906-11-01), DE, pages 3905 - 3912, XP055960706, ISSN: 0365-9496, Retrieved from the Internet <URL:https://onlinelibrary.wiley.com/doi/pdf/10.1002/cber.19060390470> DOI: 10.1002/cber.19060390470**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **O'BRIEN NATHAN J. ET AL: "In Situ Activation of an Indium(III) Triazenide Precursor for Epitaxial Growth of Indium Nitride by Atomic Layer Deposition", CHEMISTRY OF MATERIALS, vol. 32, no. 11, 24 April 2020 (2020-04-24), US, pages 4481 - 4489, XP093119777, ISSN: 0897-4756, Retrieved from the Internet <URL:http://pubs.acs.org/doi/pdf/10.1021/acs.chemmater.9b05171> DOI: 10.1021/acs.chemmater.9b05171**
- **ROUF POLLA ET AL: "Hexacoordinated Gallium(III) Triazenide Precursor for Epitaxial Gallium Nitride by Atomic Layer Deposition", CHEMISTRY OF MATERIALS, vol. 33, no. 9, 22 April 2021 (2021-04-22), US, pages 3266 - 3275, XP093119778, ISSN: 0897-4756, Retrieved from the Internet <URL:https://pubs.acs.org/doi/pdf/10.1021/acs.chemmater.1c00244> DOI: 10.1021/acs.chemmater.1c00244**
- **OUSSAMA M. EL-KADRI ET AL: "Film growth precursor development for metal nitrides. Synthesis, structure, and volatility of molybdenum(vi) and tungsten(vi) complexes containing bis(imido)metal fragments and various nitrogen donor ligands", DALTON TRANSACTIONS, no. 16, 1 January 2006 (2006-01-01), GB, pages 1943, XP055472547, ISSN: 1477-9226, DOI: 10.1039/b515378f**
- **KHALED SOUSSI ET AL: "Asymmetrically substituted triazenes as poor electron donor ligands in the precursor chemistry of iron(II) for iron-based metallic and intermetallic nanocrystals", DALTON TRANSACTIONS, vol. 46, no. 38, 1 January 2017 (2017-01-01), GB, pages 13055 - 13064, XP055472556, ISSN: 1477-9226, DOI: 10.1039/C7DT02755A**
- **DIRK PFEIFFER ET AL: "Synthesis, structure, and characterization of lanthanide complexes bearing 1,3-diphenyltriazenido ligands", JOURNAL OF ORGANOMETALLIC CHEMISTRY., vol. 588, no. 2, 1 October 1999 (1999-10-01), CH, pages 167 - 175, XP055472632, ISSN: 0022-328X, DOI: 10.1016/S0022-328X(99)00371-X**
- **E G RUKHADZE ET AL: "Triazene chelates. III. Chelates of alkyl aryl triazenes", ZHURNAL OBSHCHEI KHIMII, vol. 39, no. 2, 1 January 1969 (1969-01-01), pages 303 - 306, XP055472646**
- **RICHARD H. SMITH ET AL: "1,3-Dialkyltriazenes: tautomeric equilibria and rates and products of decomposition", JOURNAL OF ORGANIC CHEMISTRY, vol. 54, no. 5, 1 March 1989 (1989-03-01), pages 1036 - 1042, XP055472559, ISSN: 0022-3263, DOI: 10.1021/jo00266a008**

## Beschreibung

Die Erfindung betrifft die Verwendung eines Metallkomplexes, der mindestens einen Liganden der Formel $R^1-N_3-R^2$ aufweist, wobei $R^1$ und $R^2$ Alkylreste mit 1 bis 12 C-Atomen sind, sind, zum Abscheiden des Metalls oder einer Verbindung des Metalls aus der Gasphase. Die Erfindung betrifft außerdem Verfahren zur Abscheidung von Metallen aus den Metallkomplexen.

Stand der Technik

Die metallorganische Gasphasenabscheidung, und insbesondere die metallorganische Gasphasenepitaxie sind wichtige Verfahren, um dünne Schichten von Metallen oder Metallverbindungen auf Substraten zu erzeugen. Diese Verfahren werden insbesondere in der Halbleiterindustrie eingesetzt. Dabei werden metallorganische Verbindungen, gegebenenfalls in Verbindung mit weiteren reaktiven Verbindungen, in Prozesskammern eingeführt, wo bei Unterdruck bis Normaldruck eine Reaktion auf der Oberfläche erwärmter Substrate stattfindet, die zur Abscheidung der Schicht führt. Mit solchen Verfahren kann eine Vielzahl von metallhaltigen Schichten, wie Halbleiterkristalle, amorphen Schichten, metallische Verbindungen oder Metallschichten, auf Substraten abgeschieden werden. Für eine Übersicht wird beispielhaft auf das "Handbook of Thin Film Deposition - Processes and Technologies", 2. Auflage 2001, Herausgeber: Krishna Seshan, Kapitel 4, S. 151-203, von J. Zilko verwiesen.

Für viele Anwendungen, insbesondere in dem Halbleiterbereich, ist es unabdingbar, dass solche metallhaltigen Schichten in hoher Reinheit erzeugt werden. Bereits geringfügige Spuren von Verunreinigungen können den bestimmungsgemäßen Gebrauch beeinträchtigen.

Zur Erzeugung hochreiner metallhaltiger Schichten sind jedoch nur sehr wenige metallorganische Vorläuferverbindungen geeignet. Dies liegt oft daran, dass eine geeignete metallorganische Vorläuferverbindung bei hoher Temperatur in die Gasphase überführbar sein sollte. Viele metallorganische Verbindungen sind jedoch thermisch instabil. Sie zersetzen sich (zerfallen) bei höheren Temperaturen und können daher nicht durch Erwärmen oder auf andere Weise in die Gasphase überführt werden. Problematisch ist dabei, dass organometallische Verbindungen oft Feststoffe sind, die relativ hohe Verdampfungstemperaturen aufweisen, bei denen bereits Zersetzungsprozesse, unter anderem wegen den reaktiven organischen Liganden, stattfinden.

Wenn eine metallorganische Verbindung verdampft werden kann, wird üblicherweise die Temperatur in der Gasphase über dem Substrat, also dem Target der Abscheidung, weiter erhöht, bis eine Zersetzung stattfindet. Jedoch sind selbst organometallische Verbindungen, die in die Gasphase überführbar sind, oft nicht zur Abscheidung von Metallen geeignet. Der Grund dafür ist, dass die Abscheidung des Metalls auf der Substratoberfläche derart erfolgen muss, dass keine noch so geringen Verunreinigungen durch Komponenten der Komplexliganden, wie Kohlenstoff, Stickstoff oder Sauerstoff, erfolgt. Auch diese Voraussetzung wird oft nicht erfüllt, weil bei den erforderlichen hohen Zerfallstemperaturen ein Gemisch von hochreaktiven Intermediaten, ionischen Resten und Radikalen entsteht, so dass auf der Substratoberfläche oft unerwünschte Nebenreaktionen stattfinden.

Zur Abscheidung von metallhaltigen Schichten in der Gasphase werden oft Metallalkylverbindungen als Vorläuferverbindungen eingesetzt. Eine Übersicht über geeignete Verbindungen ist in der oben zitierten Publikation von J. Zilko enthalten. Allerdings weisen die Alkylverbindungen vieler Metalle oft Eigenschaften auf, welche die Eignung für Abscheidungsreaktionen in der Gasphase beeinträchtigen. Dabei ist zum einen nachteilig, dass viele reine Metallalkylverbindungen der Übergangsmetalle instabil sind, zum Zerfall neigen und nicht lagerfähig sind. Zum anderen neigen sie dazu, dass anionischer Kohlenstoff, der am Übergangsmetall gebunden ist, in unerwünschter Weise mit in die Schicht eingebaut wird. Sie weisen oft auch relativ hohe Verdampfungstemperaturen auf, was für Anwendungen bei der Gasphasenabscheidung nachteilig ist.

Es besteht daher ein kontinuierlicher Bedarf nach verbesserten Verfahren und Materialien zur Abscheidung von Metallen und metallhaltiger Schichten durch metallorganische Gasphasenabscheidung.

Im Stand der Technik sind metallorganische Komplexe bekannt, die N-Aryl- und N,N'-Diaryl-Triazenidoliganden aufweisen. Allerdings wird im Stand der Technik nicht offenbart, solche Komplexe zur Abscheidung von Metallen aus der Gasphase zu nutzen. Dies ist nicht überraschend, weil solche Metallkomplexe mit diesen Liganden nicht eine ausreichende Flüchtigkeit und Zersetzbarkeit besitzen, die für die Verwendung bei der Abscheidung von Metallen aus der Gasphase zwingend erforderlich ist. Daher würden bei solchen Reaktionen nichtflüchtige Kohlenstoff-Verunreinigungen erhalten.

Metallorganische Komplexe mit Triazenidoliganden, die mit aromatischen Resten substituiert sind, werden in einer Vielzahl von Publikationen beschrieben. Allerdings betreffen nahezu alle diese Publikationen lediglich die Synthese und Struktur solcher Komplexe, während praktische Anwendungen der Verbindungen nur in Ausnahmefällen beschrieben werden.

Dabei sind Methoden bekannt, um verschieden substituierte Vorläuferverbindungen für Triazenid-Anionen bereitzustellen. Im Jahr 1981 beschrieben Brand und Roberts die elektronische Konfiguration von 1,3-Dialkyltriazenyl-Radikalen und erwähnten in einer Fußnote, dass sich Di-*tert*-Butyltriazen ausgehend von *tert*-Butylazid und *tert*-Butyllithium darstellen lässt. Weder experimentelle noch spektroskopische Daten wurden veröffentlicht.[1] Ethyl- und methylsubstituierte Triazene wurden zudem 1983 von Smith und Michejda beschrieben, wobei die Synthesen entweder mit Methyllithium oder dem Grignard-Reagenz Ethylmagnesiumbromid durchgeführt wurden.[2] Darüber hinaus wurden Synthesen unterschiedlicher Alkyl- und Aryl-substituierten Triazene und Untersuchungen zu tautomeren Gleichgewichten in verschiedenen Veröffentlichungen diskutiert.[3,4]

Erdalkali- und Alkalimetall-Verbindungen der Triazene sind ausschließlich von Aryl-substituierten Systemen bekannt. Bei kristallographisch charakterisierten Verbindungen handelt es sich in allen Publikationen um solvatisierte Verbindungen bzw. Verbindungen, in welchen das entsprechende Metall mithilfe von TMEDA oder [15]-Krone-5 stabilisiert wird.[6-10]

Triazenido-Verbindungen mit Elementen der dritten Hauptgruppe sind seit mehreren Jahrzenten literaturbekannt.

Das US-Patent 3,386,985 beschreibt verschiedene metallorganische Verbindungen mit, die verschiedene Triazenidoliganden, unter anderem 1,3-Dimethyltriazen, aufweisen. Es wird vorgeschlagen, die Verbindungen als Kettentransfer-Reagentien oder Inhibitoren in Polymerisationsverfahren einzusetzen. Verfahren zur Abscheidung von Metallen werden nicht beschrieben.

Brinckman et al.[11] beschreiben metallorganische Komplexe mit 1,3-Dimethyltriazenid als Liganden, wobei die Ergebnisse weitgehend mit den in US3,386,985 enthaltenen übereinstimmen. Zu praktischen Verwendungen der Komplexe werden keine Ausführungen gemacht. Die Ausbeuten bei den Herstellungsverfahren liegen bei maximal 76% und sind daher noch verbesserungsbedürftig.

Des Weiteren wurden in der Literatur verschiedene Komplexe, sowohl homoleptische, als auch heteroleptische, mit einem bzw. zwei Aryl-Triazenido-Liganden beschrieben.[12-16]

Im Bereich der Übergangsmetalle ist eine Vielzahl an Triazenido-Verbindungen mit aromatisch substituierten Liganden bekannt, wobei als Zentralatom frühe Übergangsmetalle wie Titan und Zirkonium als auch späte Übergangsmetalle wie Silber und Kupfer untersucht wurden.[6,17-23] Sowohl homoleptische als auch heteroleptische, molekulare oder kationische Cobalt-Komplexe mit aromatisch substituierten Triazenido-Liganden wurden in Zusammenhang mit ESR-Messungen beschrieben. Ebenso wurde von Ruthenium-Verbindungen, in Zusammenhang mit theoretischen Berechnungen, berichtet.[24-30]

Soussi et al.[5] beschreiben die Herstellung metallorganischer Eisenkomplexe, die als Liganden Alkyltriazenide, sowie zur Stabilisierung TMEDA-Liganden (Tetramethylethylendiamin) aufweisen. Die Herstellung der Komplexe erfolgt in einem relativ aufwändigen Verfahren ausgehend von $Fe[N(SiMe_3)_2]_2$ über die Route des Ligandenaustausches. Die Metallkomplexe werden zur Herstellung von intermetallischen Nanopartikeln verwendet. Das Verdampfungs- und Zersetzungsverhalten der Eisenkomplexe bei Erhöhung der Temperatur wird durch thermogravimetrische Analyse untersucht. Dabei wurde gefunden, dass die vier Metallkomplexe eine geringe thermische Stabilität aufweisen, so dass die Komplexe nicht verdampft werden können. Bei Erhöhung der Temperatur auf mehr als 200°C verbleiben feste Rückstände, die etwa 20 bis 40 Gew.% der Einwaage ausmachen. Zudem zeigt die Gewichtskurve bei Erhöhung der Temperatur auf bis zu 200°C einen unregelmäßigen Verlauf, was typisch für chemische Zerfallsreaktionen ist. Daher sind die Metallkomplexe nicht als Vorläuferverbindungen zur Herstellung von metallhaltigen Beschichtungen aus der Gasphase geeignet.

Oussama M. El-Kadri et al., DALTON TRANSACTIONS, Nr 16, 2006-01-01, S. 1943-1953 zeigt einen Wolfram komplex mit einem Liganden der Formel $R^1$-$N_3$-$R^2$ wobei $R^1$ und $R^2$ Phenylreste sind und der Wolframkomplex zum Abscheiden des Metalls oder einer Verbindung des Metalls aus der Gasphase nicht ausreichend verdampfbar ist.

Dirk Pfeiffer et al., J. of Organomet. Chem., vol. 588, no. 2, 1999-10-01, pp. 167-175 zeigt Lanthanidkomplexe mit einem Liganden der Formel $R^1$-$N_3$-$R^2$ wobei $R^1$ und $R^2$ Phenylreste sind.

E. G. Rukhadze et al., Zhurnal Obshchei Khimii, vol. 39, no. 2, 1969-01-01, pp. 303-306 zeigt Komplexe von Silber und Quecksilber mit einem Liganden der Formel $R^1$-$N_3$-$R^2$ wobei $R^1$ ein tert.-Butylrest und $R^2$ ein Phenylrest ist.

Verschiedene Seltenerdmetall-Komplexe sind zwar literaturbekannt, jedoch lediglich mit sterisch sehr anspruchsvollen Aryl-Triazenido-Liganden.[31-33]

Es besteht im Stand der Technik ein kontinuierlicher Bedarf nach Verfahren und Verbindungen, die zur Abscheidung von Metallen und metallhaltigen Schichten in der Gasphase verwendbar sind, welche die oben beschriebenen Nachteile überwinden.

## Aufgabe der Erfindung

Der Erfindung liegt die Aufgabe zugrunde, Verfahren und Verbindungen bereitzustellen, welche die oben beschriebenen Nachteile überwinden. Der Erfindung liegt insbesondere die Aufgabe zugrunde, neue und verbesserte Verbindungen bereitzustellen, um Metalle und metallhaltige Schichten aus der Gasphase abzuscheiden.

Die Verbindungen sollen eine relativ hohe Stabilität aufweisen. Dabei sollen die Verbindungen einen hohen

Dampfdruck und gleichzeitig einen niedrigen Zersetzungspunkt aufweisen. Dabei soll es möglich sein, die Verbindungen in die Gasphase zu überführen, ohne dass eine wesentliche Zersetzung stattfindet. Nach Überführung in die Gasphase, also bei Temperaturen oberhalb der Sublimationstemperatur oder Verdampfungstemperatur, sollen sich die Verbindungen zersetzen. Bevorzugt soll die Zersetzung dabei bei einer Temperatur erfolgen, die nur wenig oberhalb der Sublimationstemperatur oder Verdampfungstemperatur liegt.

Die Verbindungen sollen allgemein bei Temperaturen, bei denen Abscheidungsverfahren aus der Gasphase üblicherweise durchgeführt werden, und dabei insbesondere von 100°C bis 300°C, eine hohe Stabilität und Volatilität aufweisen. Insbesondere sollen die Verbindungen sublimierbar sein.

Der Erfindung liegt insbesondere die Aufgabe zugrunde, Verbindungen bereitzustellen, die bei Temperaturen bis zu 100°C stabil in die Gasphase überführt werden können, und die bei höheren Temperaturen, beispielsweise im Bereich von 100°C bis 400°C, zerfallen.

Die Verbindungen sollen es ermöglichen, verschiedene Metalle und metallhaltige Verbindungen aus der Gasphase abzuscheiden. Die Beschichtungen sollen hochrein sein. Insbesondere soll bei üblichen Abscheidungsverfahren kein unerwünschter Einbau von Kohlenstoff, Sauerstoff oder Stickstoff in die Beschichtungen stattfinden.

Der Erfindung liegt außerdem die Aufgabe zugrunde, möglichst einfache und effiziente Verfahren zur Herstellung solcher Verbindungen bereitzustellen. Die eingesetzten Reagenzien sollen möglichst leicht zugänglich und sicher handhabbar sein. Die Verfahren sollen mit hohen Ausbeuten durchführbar sein. Die Verfahren sollen in möglichst wenig Stufen zu den gewünschten Produkten führen und sollen unter möglichst sanften Reaktionsbedingungen durchführbar sein. Es sollen auch Verbindungen bereitgestellt werden, aus denen sich die flüchtigen Vorläuferverbindungen auf möglichst einfache Weise herstellen lassen.

## Offenbarung der Erfindung

Überraschenderweise wird die der Erfindung zugrunde liegende Aufgabe gelöst durch die Verwendungen und Verfahren gemäß den Patentansprüchen.

Gegenstand der Erfindung ist die Verwendung eines Metallkomplexes der Formel (2):

$$M[(L^1)_aX_d] \qquad (2)$$

wobei

$L^1$ die Formel $R^1$-$N_3$-$R^2$ aufweist, wobei $R^1$ und $R^2$ Alkylreste mit 1 bis 12 C-Atomen sind,
X ausgewählt ist aus H, Halogen, CO und Alkyl mit 1 bis 6 C-Atomen,
a = 2 oder 3 ist,
d = 0 oder 1 ist, und

M ausgewählt ist aus In, Co, Cu, Al, Ga, Tl und La zum Abscheiden des Metalls oder einer Verbindung des Metalls aus der Gasphase.

Dabei wird aus der Gasphase eine feste Schicht auf einem Substrat abgeschieden, die aus dem Metall besteht oder dieses enthält. Der Metallkomplex wird dabei als metallorganische Vorläuferverbindung (Precursor) eingesetzt. Das Metall aus dem Metallkomplex wird in elementarer Form oder gezielt in Form einer Verbindung des Metalls abgeschieden. So kann beispielsweise Stickstoff aus dem Liganden oder aus einem geeigneten Reaktionspartner in eine Schicht eingebaut werden, so dass ein Nitrid des abgeschiedenen Metalls entsteht. Bevorzugt wird der Metallkomplex dabei zunächst in die Gasphase überführt, oder es werden zumindest flüchtige Zwischenprodukte in die Gasphase überführt, die das Metall enthalten. In der Gasphase wird die Temperatur bei reduziertem Druck üblicherweise weiter erhöht, so dass eine (weitere) Zersetzung des Metallkomplexes stattfindet. Der Zerfall kann auch auf andere Weise angeregt oder unterstützt werden, beispielsweise durch Strahlung. Die entscheidende Zersetzung des Metallkomplexes sollte erst nach der Überführung in die Gasphase stattfinden. Auf diese Weise wird gewährleistet, dass der Metallkomplex möglichst quantitativ in der Gasphase vorliegt und zur Abscheidung des Metalls oder einer Verbindung des Metalls genutzt werden kann. Außerdem kann regelmäßig die Zahl der unerwünschten Nebenreaktionen verringert werden, wenn ein kontrollierter Zerfall erst in der Gasphase erfolgt.

In einer bevorzugten Ausführungsform weist der Metallkomplex mindestens einen Liganden **L** der Formel $R^1$-$N_3$-$R^2$ und mindestens einen weiteren Liganden **X** auf. Der weitere Ligand X ist bevorzugt ausgewählt aus Halogen, H, CO und Alkyl mit 1 bis 6 C-Atomen. Das Halogen kann F, Cl, Br oder I sein, besonders bevorzugt ist Cl. Wenn X = H ist, liegt eine Hydridokomplex vor. Dieser Komplex kann einen Liganden X aufweisen.

Der Metallkomplex weist die Formel (2) auf:

$$M[(L^1)_aX_d] \qquad (2)$$

wobei

$L^1$ die Formel $R^1$-$N_3$-$R^2$ aufweist, wobei $R^1$ und $R^2$ Alkylreste mit 1 bis 12 C-Atomen sind,
X ausgewählt ist aus H, Halogen, CO und Alkyl mit 1 bis 6 C-Atomen,
a = 2 oder 3 ist,
d = 0 oder 1 ist, und
M ausgewählt ist aus In, Co, Cu, Al, Ga, Tl und La.

Die Alkylreste $R^1$ und $R^2$ können insbesondere 1 bis 6 C-Atome oder 1 bis 4 C-Atome aufweisen und können verzweigte Alkylreste sein.

Insbesondere können $R^1$ und $R^2$ ausgewählt sein ausgewählt aus Methyl, Ethyl, Propyl, Isopropyl, *tert*-Butyl und n-Propyl.

Insbesondere können alle oder ein Teil der Reste $R^1$ und $R^2$ *tert*-Butyl sein.

Die Metallkomplexe können homoleptisch sein und ausschließlich Liganden $L^1$ der Formel $R^1$-$N_3$-$R^2$ aufweisen oder heteroleptisch sein.

$R^1$ und $R^2$ können auch gleich sein.

In einer Ausführungsform kann der Metallkomplex mindestens einen Liganden $L^1$ enthalten, der *tert*-Butyl-$N_3$-*tert*-Butyl oder *tert*-Butyl-$N_3$-Methyl ist.

In einer weiteren Ausführungsform können in dem Metallkomplex alle Liganden $L^1$ der *tert*-Butyl-$N_3$-*tert*-Butyl oder *tert*-Butyl-$N_3$-Methyl sein.

In der erfindungsgemäßen Verwendung kann der Metallkomplex sublimierbar oder verdampfbar sein, ohne dass eine Zersetzung erfolgt.

Vorteilhaft ist das Molekulargewicht des Metallkomplexes kleiner kleiner als 600 g/Mol.

In der Verwendung gemäß der Erfindung ist der Metallkomplex vorteilhaft bei 100°C thermisch stabil und/oder zerfällt in einem Temperaturbereich von 100°C bis 400°C, wobei die Temperatur bei Normaldruck oder bei reduziertem Druck im Bereich von $10^{-3}$ bis 900 mbar ermittelt wird.

In der Verwendung gemäß der Erfindung erfolgt vorteilhaft das Abscheiden des Metalls oder der Verbindung des Metalls aus der Gasphase bei reduziertem Druck im Bereich von $10^{-3}$ bis 900 mbar, bevorzugt im Bereich von $10^{-2}$ bis 1 mbar, und insbesondere $10^{-2}$ mbar.

In einer bevorzugten Ausführungsform weist der Metallkomplex eine der Formeln (3) bis (5) auf:

$$M[(L^1)_2X] \qquad (3)$$

wobei

$L^1$ die Formel $R^1$-$N_3$-$R^2$ aufweist, wobei $R^1$ und $R^2$ Alkylreste mit 1 bis 12 C-Atomen sind,
X ausgewählt ist aus H und Alkyl mit 1 bis 6 C-Atomen, und
M = Al oder Ga ist;

$$M[(L^1)_3] \qquad (4)$$

wobei

$L^1$ die Formel $R^1$-$N_3$-$R^2$ aufweist, wobei $R^1$ und $R^2$ Alkylreste mit 1 bis 12 C-Atomen sind, und
M = In, Tl oder La ist;

$$M_x(L^1)_a \qquad (5)$$

wobei

$L^1$ die Formel $R^1$-$N_3$-$R^2$ aufweist, wobei $R^1$ und $R^2$ Alkylreste mit 1 bis 12 C-Atomen sind,
x eine ganze Zahl zwischen 1 und 4 ist, und
a eine ganze Zahl zwischen 2 und 8 ist, mit der Maßgabe, dass a/x = 1 oder 2 ist, und

M = Co oder Cu ist.

Dabei ist insbesondere das Metall Co, wenn a/x = 2 ist, und das Metall Cu, wenn a/x = 1 ist.

In einer bevorzugten Ausführungsform ist der Metallkomplex bei 25°C und Normaldruck (1013 mbar) flüssig. Flüssigkeiten sind besonders gut für solche Verfahren geeignet, weil der Übergang in die Gasphase bereits bei niedriger Temperatur erfolgt, und weil Flüssigkeiten besonders gut handhabbar sind. In einer bevorzugten Ausführungsform ist der Metallkomplex bei 25°C und Normaldruck fest und weist eine relativ niedrige Verdampfungstemperatur auf.

Bevorzugt weist der Metallkomplex eine Sublimations- oder Verdampfungstemperatur bei Normaldruck auf, die kleiner als 120°C ist, insbesondere kleiner als 100°C.

Bevorzugt erfolgt der Übergang in die Gasphase durch Verdampfung oder Sublimation bei einer Temperatur, die kleiner als 120°C ist, insbesondere kleiner als 100°C. Die Verdampfung oder Sublimation erfolgt dabei bevorzugt bei reduziertem Druck im Bereich von $10^{-3}$ bis 900 mbar, bevorzugt im Bereich von $10^{-2}$ bis 1 mbar, und insbesondere bei $10^{-2}$ mbar.

Allgemein wird der Druck bei dem erfindungsgemäßen Verfahren bevorzugt in Bereichen eingestellt, die für solche Verfahren üblich sind. Somit werden das Verfahren und die Verwendung bevorzugt bei einem Druck im Bereich von $10^{-3}$ bis 900 mbar, und besonders bevorzugt im Bereich von $10^{-2}$ bis 1 mbar durchgeführt, und insbesondere bei $10^{-2}$ mbar. Im Rahmen dieser Anmeldung werden Parameter bei der Reaktion in der Gasphase, wie die Verdampfungs-, Sublimations- oder Zersetzungstemperaturen, oder Temperaturen, bei denen Verbindungen stabil sind, bevorzugt bei den reduzierten Drücken gemessen, die bei solchen Reaktionen eingestellt werden, und dabei insbesondere bei einem Druck von $10^{-2}$ mbar.

In einer bevorzugten Ausführungsform ist der Metallkomplex bei Normaldruck und/oder bei reduziertem Druck in der Gasphase sublimierbar oder verdampfbar, ohne dass eine Zersetzung (ein Zerfall) erfolgt. Die bedeutet beispielsweise, dass bis zu 90% Gew.%, bis zu 95% Gew.% oder bis zu 98% Gew.% in die Gasphase überführt werden können, ohne dass eine Zersetzung erfolgt. Der Zerfall lässt sich nach üblichen Methoden verfolgen, insbesondere SDTA (Simultaneous differential thermal analysis).

Bevorzugt ist der Metallkomplex bei Normaldruck, aber insbesondere bei reduziertem Druck in der Gasphase bei 100°C, besonders bevorzugt bei 120°C oder 150°C, thermisch stabil. Dies bedeutet, dass bei einer solchen Temperatur noch keine wesentliche Zersetzung einsetzt, oder dass zumindest nicht mehr als 5 Gew.%, insbesondere nicht mehr als 2 Gew.% oder nicht mehr als 1 Gew.% zersetzt werden. Bevorzugt geht der Metallkomplex unterhalb dieser Temperatur in die Gasphase über. Dabei liegt der reduzierte Druck im Bereich von $10^{-3}$ bis 900 mbar, bevorzugt im Bereich von $10^{-2}$ bis 1 mbar, und insbesondere bei $10^{-2}$ mbar.

In einer bevorzugten Ausführungsform weist der Metallkomplex ein relativ niedriges Molekulargewicht auf. Relativ leichte Komplexe können oft besser in die Gasphase überführt werden. Die im Rahmen dieser Anmeldung beschriebenen Metallkomplexe können in vorteilhafter Weise mit Liganden hergestellt werden, die ein relativ geringes Molekulargewicht aufweisen. Das Molekulargewicht des Metallkomplexes ist bevorzugt kleiner als 600 g/Mol, besonders bevorzugt kleiner als 450 g/Mol, und ganz besonders bevorzugt kleiner als 350 g/Mol.

Gegenstand der Erfindung ist auch ein Verfahren zur Herstellung von beschichteten Substraten, umfassend die Schritte:

(a) Bereitstellen eines wie oben beschriebenen Metallkomplexes, und
(b) Abscheiden des Metalls oder einer Verbindung des Metalls auf der Oberfläche des Substrats durch metallorganische Gasphasenabscheidung.

In einer bevorzugten Ausführungsform ist das Verfahren eine metallorganische chemische Gasphasenabscheidung (MOCVD). Die metallorganische Gasphasenabscheidung (MOCVD, "Metallo-Organic Chemical Vapor Deposition") ist ein Beschichtungsverfahren aus der Gruppe der chemischen Gasphasenabscheidung (CVD), bei dem die Abscheidung einer festen Schicht auf ein Substrat aus der chemischen Dampfphase unter Einsatz einer metallorganischen Vorläuferverbindung (Precursor) erfolgt.

In einer bevorzugten Ausführungsform ist das Verfahren eine metallorganische chemische Gasphasenepitaxie (MOVPE; für "Metal Organic Vapor Phase Epitaxy", auch "Organometallic Vapor Phase Epitaxy", OMVPE). Während durch MOCVD jede Abscheidung auf einem Substrat möglich ist, ist MOVPE ein Epitaxie-Verfahren und betrifft somit das kristalline Wachstum auf einem kristallinen Substrat. Die Verfahren, insbesondere MOVPE, werden insbesondere zur Abscheidung von halbleitenden Materialien verwendet.

Mit den erfindungsgemäßen Verwendungen und Verfahren wird das Metall oder eine Verbindung des Metalls aus der Gasphase abgeschieden. Dabei erfolgt die Abscheidung auf einem Substrat. Die metallhaltige Abscheidung wird dabei im Allgemeinen als Schicht oder Beschichtung bezeichnet. Die Verbindung des Metalls ist bevorzugt ausgewählt aus Halbleiterverbindungen, Legierungen, Nitriden, Phosphiden, Arseniden und Siliziden. Solche Verbindungen werden nach bekannten Verfahren durch Zusatz von weiteren Verbindungen in der Gasphase erhalten, die mit dem

Metall unter den Bedingungen in der Vorrichtung reagieren können. Für eine Übersicht wird ebenfalls auf die oben zitierte Publikation von J. Zilko verwiesen.

Verfahren der metallorganischen Gasphasenabscheidung und der metallorganischen Gasphasenepitaxie werden bevorzugt wie nachfolgend beschrieben durchgeführt. Die Herstellung der metallhaltigen Schichten erfolgt in einer Reaktionskammer. In dieser liegt ein zu beschichtendes Substrat vor, das auf eine erhöhte Temperatur geheizt wird. Ein Gasstrom mit dem Metallkomplex als Vorläuferverbindung und üblicherweise einem Trägergas wird in die Reaktionskammer eingeführt, wo die Vorläuferverbindung in der Gasphase vorzerlegt und radikalische Gruppen sich auf dem Substrat anlagern. Thermisch aktiviert haben radikalische Gruppen eine gewisse Bewegungsfreiheit auf dem Substrat, bis sich das Metallatom an geeigneter Stelle in die Schicht einbaut. Der organische Rest wird mit elementarem Wasserstoff gesättigt, so dass eine volatile organische Verbindung entsteht. Das Restgas wird aus der Reaktionskammer abgeführt. Es ist bevorzugt, dass der Metallkomplex vor Einführung in die Reaktionskammer in die Gasphase überführt wird. In einer weiteren Ausführungsform ist es auch denkbar, eine Flüssigkeit, die den Metallkomplex enthält, erst bei Einführung in die Reaktionskammer zu verdampfen.

Überraschenderweise wurde gefunden, dass die im Rahmen dieser Anmeldung beschriebenen Metallkomplexe in hohem Maße als Vorläuferverbindungen zur Abscheidung von Metallen aus der Gasphase geeignet sind. Sie vereinen dabei überraschenderweise mehrere vorteilhafte Eigenschaften, die für solche Verfahren erforderlich sind. Sie sind in hohem Maße stabil, volatil und können so eingesetzt werden, dass bei der Abscheidung des Metalls oder einer Verbindung des Metalls auf einem Substrat nahezu keine Verunreinigungen entstehen.

Bevorzugt wird der Metallkomplex bei dem Verfahren sublimiert oder verdampft, ohne dass eine Zersetzung erfolgt. Während bei der Sublimation ein Feststoff vorgelegt wird, wird bei der Verdampfung eine Flüssigkeit oder eine Lösung vorgelegt. Bevorzugt erfolgt also während der Überführung des Metallkomplexes in die Gasphase kein Zerfall oder kein wesentlicher Zerfall. Der Zerfallsprozess lässt sich nach üblichen Methoden verfolgen, insbesondere SDTA (Simultaneous differential thermal analysis). Die relevanten Eigenschaften eines Metallkomplexes bei Erhöhung der Temperatur lassen sich durch Thermogravimetrische Analyse (TGA) bestimmen, die bei normalem Druck (1013 mbar) oder auch bei reduziertem Druck durchführbar ist. Dabei ist es bevorzugt, dass die Kurve Menge/Temperatur (bei gleichmäßiger T-Erhöhung, z. B. 5 oder 10 K/min) einen gleichmäßigen Verlauf zeigt. Eine Sublimation liegt dabei im Allgemeinen vor, wenn bei oder ab einer Sublimationstemperatur eine schnelle Gewichtsabnahme erfolgt, und dabei kein oder nur wenig Rückstand verbleibt. Bevorzugt zeigt die TGA-Kurve keine wesentlichen Unregelmäßigkeiten, wie Höcker oder Dellen.

Bevorzugt erfolgt die Überführung des Metallkomplexes in die Gasphase möglichst vollständig. Dabei ist es bevorzugt, dass bei der TGA bei Normaldruck und/oder reduziertem Druck ein möglichst geringer Rückstand verbleibt, beispielsweise weniger als 20 Gew.-%, weniger als 10 Gew.%, weniger als 5 Gew.-%, oder insbesondere weniger als 3 Gew.%, bezogen auf die eingesetzte Menge des Metallkomplexes. Der Rückstand entspricht dem Plateauwert, an dass sich das das Gewicht des Rückstandes bei weiterer T-Erhöhung auf beispielsweise bis zu 500°C, 600°C oder 700°C annähert. Wenn das Gewicht des Rückstandes (deutlich) unterhalb dem Gewicht des Metalls in der vorgelegten Verbindung liegt, deutet dies auf eine Sublimation oder Verdampfung der Verbindung hin. Bevorzugt wird bei der TGA mindestens 50 Gew.%, insbesondere mindestens 80 Gew.% oder mindestens 90 Gew.% des Metalls in die Gasphase überführt. Allgemein ist es aus Effizienzgründen vorteilhaft, wenn das Metall quantitativ verdampft wird.

Es wurde gefunden, dass die erfindungsgemäßen Metallkomplexe relativ stabil sind. Sie sind bevorzugt bei Raumtemperatur stabil und dadurch beliebig lange haltbar. In einer bevorzugten Ausführungsform ist der Metallkomplex bei Temperaturen bis zu 100°C thermisch stabil.

Bevorzugt erfolgt bei der TGA bei Normaldruck ein Massenverlust von 3% erst bei einer Temperatur oberhalb 80°C, bevorzugt oberhalb 100°C, und besonders bevorzugt oberhalb 120°C. Bevorzugt erfolgt der Massenverlust von 3% bei einer Temperatur zwischen 80°C und 220°C, insbesondere zwischen 80°C und 160°C.

Bevorzugt zerfällt der Metallkomplex unter Bedingungen der Gasphasenabscheidung, die üblicherweise bei reduziertem Druck durchgeführt wird, bei Temperaturen zwischen 100°C und 400°C, insbesondere zwischen 120°C und 300°C, oder zwischen 150°C und 250°C. Bevorzugt liegt der Komplex bei diesen Temperaturen in der Gasphase vor. Dabei liegt der reduzierte Druck im Bereich von $10^{-3}$ bis 900 mbar, bevorzugt im Bereich von $10^{-2}$ bis 1 mbar, und insbesondere bei $10^{-2}$ mbar. Der Zerfall erfolgt oft in einer exothermen Reaktion. Der Zerfall in diesem Bereich ist bevorzugt vollständig.

Bevorzugt wird der Metallkomplex erst nach Überführung in die Gasphase zersetzt. Bevorzugt erfolgt somit die Zersetzung bei einer Temperatur, die oberhalb der Sublimationstemperatur oder Verdampfungstemperatur liegt. Bevorzugt liegt die Zersetzungstemperatur bei nicht mehr als 200°C, nicht mehr als 100°C, oder bevorzugt nicht mehr als 50°C oberhalb der Sublimationstemperatur oder Verdampfungstemperatur, insbesondere bei reduziertem Druck wie oben angegeben. Bevorzugt ist der Metallkomplex bei dieser Zersetzungstemperatur vollständig, oder zu 50%, zersetzt. Wenn die Zersetzung bei einer Temperatur erfolgt, die nicht allzu weit oberhalb der Sublimation- oder Verdampfungstemperatur liegt, kann die Abscheidung in der Gasphase oft besonders effizient durchführbar sein.

Bevorzugt wird bei der Abscheidung aus der Gasphase das elementare Metall oder die gewünschte Verbindung

des Metalls in Reinform erhalten. Dies bedeutet, dass keine oder vernachlässigbare Verunreinigungen bestimmt werden können, beispielsweise von weniger als 500 ppb, weniger als 100 ppb oder weniger als 10 ppb. Die Reinheit des abgeschiedenen Metalls oder der metallhaltigen Verbindung kann mittels üblicher Methoden bestimmt werden, beispielsweise durch Sekundärionenmassenspektrometrie.

In einer weiteren bevorzugten Ausführungsform enthält die Beschichtung mindestens eine weitere atomare Komponente, insbesondere mindestens ein weiteres Metall, oder ein Element, ausgewählt aus Si, N, P oder As. So ist es nach bekannten Methoden möglich, weitere Metalle oder Elemente in die Beschichtung einzuarbeiten, um entsprechende Legierungen oder Verbindungen zu erhalten.

1. Zur Herstellung der Metallkomplexe der

Formel (2) eingesetzt wird ein Verfahren zur Herstellung des erfindungsgemäß verwendbaren Metallkomplexes, umfassend die Schritte

(A) Bereitstellen einer Verbindung der Formel $R^1$-($N_3$)A-$R^2$, wobei A ausgewählt ist aus H oder einem Alkalimetall, insbesondere Li, Na oder K, besonders bevorzugt Li, und
(B) in Kontakt bringen mit einer Verbindung des Metalls.

Dieses Verfahren ist nicht Bestandteil der Erfindung.

Die Verbindung der Formel $R^1$-($N_3$)A-$R^2$ reagiert dann mit der Verbindung des Metalls zu dem Metallkomplex. Bevorzugt erfolgt diese Reaktion in einem einzigen Schritt. Dabei ist A bevorzugt Alkali, insbesondere Li. Die Metallkomplexe und insbesondere die Reste $R^1$, $R^2$ und A sind dabei ausgewählt wie oben beschrieben.

Bevorzugt ist dabei die in Schritt (B) eingesetzte Verbindung des Metalls ein Metallsalz, eine metallorganische Verbindung oder ein anderer Metallkomplex des Metalls. Dabei weist der andere Metallkomplex bevorzugt keinen Triazenidoliganden auf, insbesondere keinen Liganden L der Formel $R^1$-$N_3$-$R^2$, bei dem $R^1$ und $R^2$ Kohlenwasserstoffreste sind.

In einer bevorzugten Ausführungsform umfasst das Verfahren zur Herstellung der Metallkomplexe vor Schritt (A) den Schritt des Herstellens der Verbindung der Formel $R^1$-($N_3$)A-$R^2$ aus den Verbindungen $R^1$-$N_3$ und $AR^2$, wobei A ein Alkalimetall ist. Dabei wird das gesamte Verfahren bevorzugt in einem einzigen Reaktionsgemisch durchgeführt (als "Eintopfreaktion").

Ein Verfahren zur Herstellung einer Verbindung der Formel $R^1$-($N_3$)A-$R^2$ ist, in einem Reaktionsgemisch, das die Verbindungen $R^1$-$N_3$ und $AR^2$ enthält, wobei A ein Alkalimetall ist, bevorzugt Li. Die Reste $R^1$, $R^2$ und A sind dabei ausgewählt wie oben beschrieben.

Die Herstellung einer Verbindung der Formel $R^1$-($N_3$)A-$R^2$ erfolgt bevorzugt in einem inerten Lösungsmittel, insbesondere einem Kohlenwasserstoff, wie Pentan oder Hexan.

In einer Variante wird die Verbindung der Formel $R^1$-($N_3$)A-$R^2$ nach der Reaktion aufgereinigt, indem die weiteren Komponenten aus dem Reaktionsgemisch verdampft werden (Methode A). Wenn A ein Alkalimetall ist und sich in Diethylether befindet, fällt das Produkt als festes und reines Diethyletheraddukt (Alkalisalz) aus und kann durch Filtration abgetrennt werden (Methode B). Das Diethyletheraddukt kann im Vakuum komplett von Ether befreit werden oder es kann, falls gewünscht, durch Hydrolyse mit Wasser in die korrespondierende Neutralverbindung (mit A = H) überführt werden.

Es wurde gefunden, dass mit dem Verfahren Metallkomplexe, sowie die Verbindungen der Formel $R^1$-($N_3$)A-$R^2$, die wesentliche Zwischenprodukte für die Komplexsynthese sind, auf relativ einfache Weise, effizient und in hoher Ausbeute herstellbar sind. Bei der Herstellung des Metallkomplexes wird dabei von einer Verbindung der Formel $R^1$-($N_3$)A-$R^2$ ausgegangen. Dabei kann A ein Wasserstoffrest H sein. Die Verbindung ist dann insgesamt neutral. Es wurde gefunden, dass mit verschiedenen Metallsalzen eine effiziente Metallkomplexbildung unter relativ einfachen Bedingungen stattfindet. So sind beispielsweise Metallkomplexe von Al, Ga und In ausgehend von diesen Verbindungen erhältlich.

Auch wurde gefunden, dass viele Metallkomplexe zugänglich sind, wenn die Verbindung der Formel $R^1$-($N_3$)A-$R^2$ ein Alkalimetallsalz ist. Dabei ist A insbesondere Li, Na oder K, und besonders bevorzugt Li. Solche Verbindungen wurden beispielsweise eingesetzt, um auf einfache Weise und mit hoher Ausbeute Salze von Co, Cu oder Ru herzustellen, was nicht Bestandteil der Erfindung ist.

Die Verbindungen der Formel $R^1$-($N_3$)A-$R^2$, wobei A = H oder ein Alkalimetall ist, sind auf einfache Weise durch Reaktion der Vorläuferverbindungen $R^1$-$N_3$ und A-$R^2$ erhältlich, wobei A bevorzugt ein Alkalimetall ist. Die Reaktion findet bevorzugt in einem organischen aprotischen Lösungsmittel statt, insbesondere in Kohlenwasserstoffen, besonders bevorzugt in Pentan oder Hexan. Dabei wird eine Alkalialkylverbindung, insbesondere eine Organo-Lithiumverbindung, bevorzugt langsam zugetropft. Bevorzugt wird das Reaktionsgemisch zunächst bei niedriger Temperatur, beispielsweise <15°C, belassen und anschließend auf Raumtemperatur erwärmt.

Bei der Reaktion zu dem Metallkomplex wird die so erhaltene Verbindung der Formel $R^1$-($N_3$)A-$R^2$, oder die gleiche Verbindung aus anderer Herkunft, in Kontakt mit einer Verbindung des Metalls mit ausreichender Reaktivität gebracht, wie einem Metallsalz oder einer organometallischen Verbindung des Metalls, insbesondere mit einem Metallhalogenid, wie $CoCl_2$ oder CuCl, oder einer organometallischen Verbindung, wie $AlMe_3$ oder $InMe_3$, oder einem Metallhydrid, wie $GaH_3$ ($OEt_2$), oder einem Vorläufermetallkomplex, wie La(HMDS)$_3$ (HMDS = Hexamethyldisilazan) oder [$RuCl_2$(p-Cymen)]$_2$. Dabei wurde gefunden, dass beispielsweise im Falle von Kobalt bereits eine Reaktion mit einem einfachen Salz ausreicht, während bei Elementen der dritten Hauptgruppe metallorganische Vorläuferverbindungen bevorzugt sind. Um Lanthan in Metallkomplexe zu überführen, wird bevorzugt von anderen Metallkomplexen ausgegangen. In allen Fällen wurde gefunden, dass ausgehend von Verbindungen der Formel $R^1$-($N^3$)A-$R^2$ stabile Komplexe mit den entsprechenden Liganden in hohen Ausbeuten erhalten werden können.

Ohne an eine Theorie gebunden zu sein, wird vermutet, dass die relativ schwache N-N-Einfachbindung in den Metallkomplexen zu einer relativ leichten thermischen Zersetzbarkeit bei relativ niedrigen Temperaturen führt. Dabei wird angenommen, dass $N_2$ als stabiles Zersetzungsprodukt entsteht, während weitere stickstoffhaltige Alkylreste als molekulare flüchtige Verbindungen gebildet werden. Alle Reaktionsprodukte sind nicht oder wenig reaktiv und sicher in der Handhabung, so dass das Verfahren auf relativ einfache Weise und mit hoher Ausbeute durchführbar ist.

Vorteilhaft ist auch, dass die Liganden, bei denen $R^1$ oder $R^2$ beispielsweise Methyl oder tert-Butyl ist, relativ niedrige Molekulargewichte aufweisen. Dies führt zu einer relativ guten Verdampfbarkeit oder sogar Sublimierbarkeit bei relativ niedrigen Temperaturen. Die Anwendung gerade dieser beiden Liganden ist, wie überraschend gefunden wurde, von besonderer Bedeutung für die Bereitstellung hochgradig flüchtiger Metallverbindungen für ALD und MOCVD. Die Synthese über das leicht zugängliche und vor allem technologisch sicher handhabbare tert-Butylazid birgt erhebliche Vorteile gegenüber etablierten 1,3-Diazaallyl-Systemen wie N,N'-Dialkylacetamido- oder N,N'-Dialkyl-N"-dialkylguanidato-Ligandensystemen. Die Zerfallstemperaturen der im folgenden beschriebenen Triazenido-Komplexe unter ALD- und CVD-Bedingungen ist niedriger, der unerwünschte Kohlenstoffeinbau weniger prägnant als in Amidinato- und Guanidinato-Komplexen.

Bevorzugt wird bei Schritt (B) eine metallorganische Verbindung eingesetzt, insbesondere eine Organolithiumverbindung, insbesondere Alkyllithium mit 1 bis 6 C-Atomen, wie Methyllithium. Bevorzugt wird die Verbindung in Ether gelöst eingesetzt. Dabei wird als Lösungsmittel bevorzugt Pentan eingesetzt. Bevorzugt wird in Schritt (B) die Verbindung des Metalls zu der Verbindung aus Schritt (A) zugegeben, insbesondere kontinuierlich, wie durch Zutropfen.

Besonders vorteilhaft ist es, wenn als Zwischenprodukt ein Salz des Liganden eingesetzt wird, insbesondere das Lithiumsalz. Bei der Herstellung solcher organischen Salze ist die Auswahl des Lösungsmittels von Bedeutung. So fällt das Lithiumsalz von Methyl-*tert*-Butyltriazen in Pentan als Lösungsmittel aus, wenn als Edukt Methyllithium in Ether gelöst eingesetzt wird. Das Lithiumsalz kann anschließend durch Filtration isoliert werden. Dagegen ist das Lithiumsalz von Di-*tert*-Butyltriazen in Pentan löslich. Es kann nach Ende der Reaktion durch Eindampfen der klaren Reaktionslösung isoliert werden. Bei beiden Synthesen fallen keine Nebenprodukte an und die Ausbeuten liegen bei über 80%. Falls gewünscht kann der Neutralligand Di-*tert*-Butyltriazen ohne weiteres durch wässrige Aufarbeitung des Reaktionsgemisches erhalten werden.

Die Lithiumsalze können vollständig getrocknet und weiterverarbeitet werden. Sie sind in unpolaren Lösungsmitteln, wie Pentan oder Toluol, löslich, aber auch in anderen gängigen Lösungsmitteln, wie Diethylether oder THF. Sie können daher in solchen Lösungsmitteln ohne weiteres in Folgereaktionen zur Herstellung der Metallkomplexe eingesetzt werden. Auch die entsprechenden Neutralliganden mit A = H, bei denen es sich um destillierbare Flüssigkeiten handelt, können in allen gängigen Lösungsmitteln für Folgereaktionen eingesetzt werden. Die Verfahren und die Zwischenprodukte ermöglichen daher eine einfache und effiziente Herstellung von Metallkomplexen in hohen Ausbeuten.

Ausgehend von den Verbindungen der Formel $R^1$-($N_3$)A-$R^2$ sind so verschiedene Komplexverbindungen über die Routen der Salz-, Alkan- (insbesondere Methan), Wasserstoff- oder Amin-Eliminierung zugänglich. Die beiden Triazenidoliganden sind dabei in der Lage, Hauptgruppenelemente der dritten oder fünften Hauptgruppe, wie Gallium, Indium oder Antimon, in homoleptischen oder heteroleptischen Verbindungen zu komplexieren. Auch die Herstellung von Komplexen mit Nebengruppenelementen, wie Co, Cu, Ru oder La, ist ohne weiteres möglich.

Wegen der einfachen Herstellung der Zwischenprodukte und Metallkomplexe, die beispielsweise in einem einzigen Reaktionsansatz erfolgen kann, und da keine problematischen Nebenprodukte auftreten, kann das Verfahren ohne weiteres hochskaliert werden und beispielsweise im Industriemaßstab durchgeführt werden. Die Metallkomplexe werden bei üblichem Erwärmen in einer Vorrichtung zur Gasphasenabscheidung, insbesondere einem "Bubbler"-Vorratsgefäß, bei Erwärmung auf Temperaturen von beispielsweise 100°C nicht zersetzt. Sie zerfallen dann, bevorzugt erst nach Übergang in die Gasphase, bei Temperaturen im Bereich von 100°C bis 400°C.

In einer bevorzugten Ausführungsform weist der Metallkomplex eine der Formeln (7) bis (12), (19), (20) sowie (104) bis (107) auf:

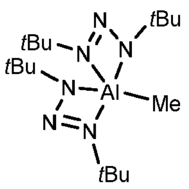

[Al(dbt)$_2$(Me)]

(7)

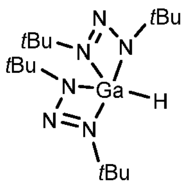

[Ga(dbt)$_2$(H)]

(8)

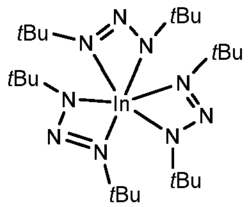

[In(dbt)$_3$]

(9)

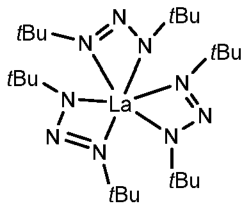

[La(dbt)$_3$]

(10)

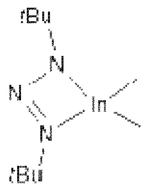

(9a)

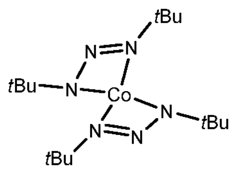

[Co(dbt)$_2$]

(11)

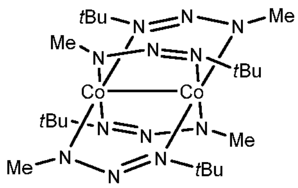

[Co(mbt)$_2$]

(12)

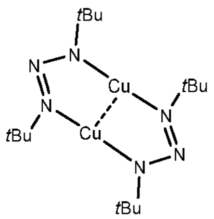

(19)

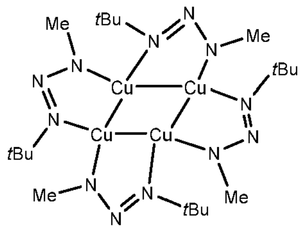

(20)

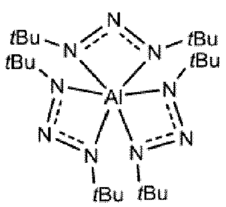

(104) [Al(dbt)$_3$]

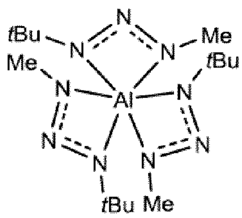

(105) [Al(mbt)$_3$]

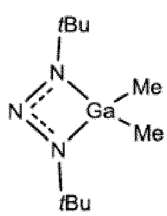

(106) [Ga(dbt)Me$_2$]

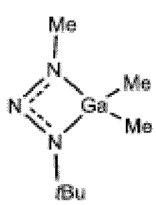

(107) [Ga(mbt)Me$_2$]

Es existieren ausserdem Verbindungen, die ausgewählt sind aus *tert*-Butyl-$(N_3)H-CH_3$ und Verbindungen der Formel $R^1-(N_3)A-R^2$, wobei $R^1$ und $R^2$ Alkylreste sind und A ein Alkali- oder Erdalkalimetall ist, insbesondere Li, Na, Cs, Ca oder K, besonders bevorzugt Li. Diese sind nicht Gegenstand der Erfindung.

Insbesondere weist die Metallverbindung eine der Formeln (21) bis (23) oder (116) bis (117) auf:

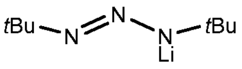
Li(dbt)

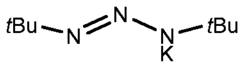
K(dbt)

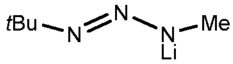
Li(mbt)

(21) (22) (23)

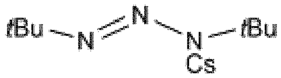

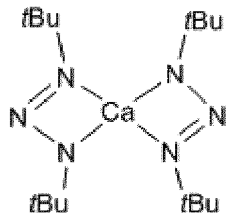

(116) [Cs(dbt)] (117) $[Ca(dbt)_2]$

Die Reste $R^1$ und $R^2$ sind dabei ausgewählt wie weiter oben beschrieben. Die Alkalimetallverbindungen sind wichtige Zwischenprodukte bei der Herstellung der Metallkomplexe. Sie können auf einfache Weise wie oben beschrieben hergestellt werden und sind auf einfache Weise aus dem Reaktionsgemisch abtrennbar. Die Verbindungen sind stabil und können durch Zugabe von Wasser in die entsprechenden Neutralverbindungen (mit A=H) überführt werden. Sie eignen sich dadurch grundsätzlich auch für andere chemische Reaktionen und die Herstellung von Komplexen für andere Anwendungen als die Abscheidung von Metallen aus der Gasphase.

Die Verwendungen, Verfahren und Verbindungen lösen die oben beschriebene Aufgabe. Es werden neue und verbesserte Verbindungen bereitgestellt, um Metalle aus der Gasphase abzuscheiden. Die Verbindungen weisen eine relativ hohe Stabilität auf. Insbesondere werden Verbindungen bereitgestellt, die einen hohen Dampfdruck und gleichzeitig einen niedrigen Zersetzungspunkt aufweisen. Dadurch können die Verbindungen in die Gasphase überführt werden, ohne dass eine wesentliche Zersetzung stattfindet. Die Verbindungen weisen insbesondere bei Temperaturen, bei denen Abscheidungsverfahren aus der Gasphase üblicherweise durchgeführt werden, und dabei insbesondere von 100°C bis 300°C, eine hohe Stabilität und Volatilität auf. Die Verbindungen ermöglichen es, verschiedene Metalle aus der Gasphase abzuscheiden, wobei die metallhaltigen Beschichtungen hochrein sind. Die Erfindung betrifft zudem einfache und effiziente Verfahren zur Herstellung solcher Verbindungen. Die eingesetzten Reagenzien sind leicht zugänglich und sicher handhabbar. Die Verfahren sind mit hohen Ausbeuten durchführbar. Sie führen in wenigen Stufen, oder sogar als Eintopfverfahren, zu den gewünschten Produkten und sind bei sanften Reaktionsbedingungen durchführbar.

Die Figuren zeigen thermogravimetrische Analysen von Metallkomplexen (TGA), die gemäß den Ausführungsbeispielen hergestellt wurden. Die TGA-Kurve zeigt, wie sich die eingesetzte Menge des Metallkomplexes mit zunehmender Temperatur verringert (in % und mg pro min und °C). Gezeigt ist jeweils auch das erste Integral der Kurve. Außerdem sind die Plateaus der Ausgangsmenge (100%) und der verbleibende Menge dargestellt, die jeweils durch eine Senkrechte bei 50% Gewichtsabnahme verbunden sind.

## Ausführungsbeispiele

Übersicht der hergestellten Verbindungen:

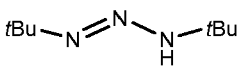
H(dbt)

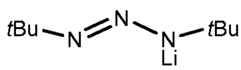
Li(dbt)

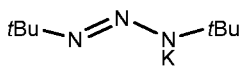
K(dbt)

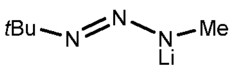

Li(mbt)

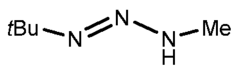

H(mbt)

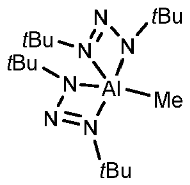

[Al(dbt)$_2$(Me)]

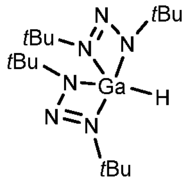

[Ga(dbt)$_2$(H)]

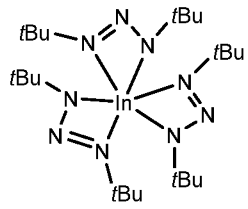

[In(dbt)$_3$]

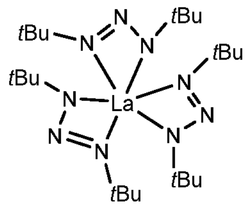

[La(dbt)$_3$]

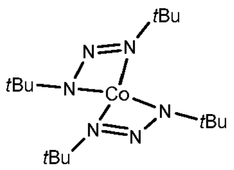

[Co(dbt)$_2$]

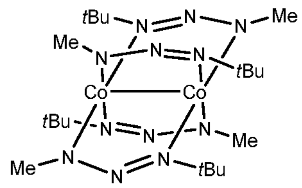

[Co(mbt)$_2$]

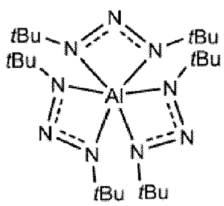

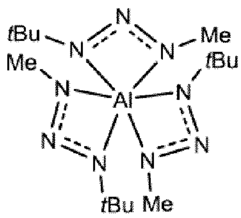

[Al(dbt)$_3$] [Al(mbt)$_3$]

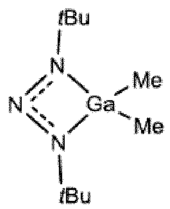

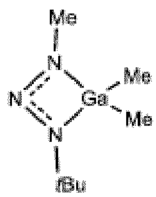

[Ga(dbt)Me$_2$] [Ga(mbt)Me$_2$]

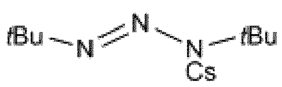

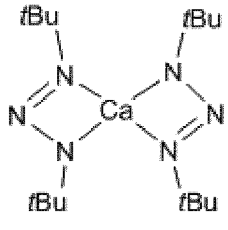

[Cs(dbt)] [Ca(dbt)$_2$]

Beispiel 1: *tert*-Butylazid

Im Allgemeinen werden organische Azide anhand zweier Kriterien als explosionsgefährlich eingestuft, wobei

es sich bei beiden Bedingungen um empirische Grenzen der Explosivität handelt.

*1)*

*[(Anzahl (N-Atome) + Anzahl (O-Atome)) / Anzahl (C-Atome)]* < 3

*2)*

*Gewichtsanteil an azidischem Stickstoff* > 25 *ω%*

Nach beiden Kriterien ist das hier beschriebene *tert*Butylazid als explosionsgefährlicher Stoff einzustufen. Unfälle bei der Darstellung oder Handhabung sind jedoch nicht bekannt. Zudem handelt es sich um eine Flüssigkeit, welche zur Aufreinigung bei 79 °C destilliert werden kann und eine $N_2$-Abspaltungstemperatur von circa 550 °C besitzt, welche für organische Azide vergleichsweise hoch ist.

Es gibt verschiedene synthetische Zugänge, von denen bereits seit Mitte der 1960er Jahre berichtet wurde. Eine Route geht beispielsweise von *tert*Butylchlorid aus, welches mit $NaN_3$ und $ZnCl_2$ als Katalysator in $CS_2$ umgesetzt wird.[34] Außerdem wird in einem weiteren synthetischen Zugang *tert*Butylnitrat mit $LiN_3$ in DMF umgesetzt.[35,36] Beide Routen können zu verschiedenen Komplikationen durch unvollständige Reaktionen oder unvollständiges Abtrennen der Nebenprodukte führen. Des Weiteren sollte der Einsatz von Kohlenstoffdisulfid aufgrund der hohen Toxizität vermieden werden. Deswegen wurde eine Route ausgehend von *tert*Butanol untersucht, bei welcher der Alkohol mit $NaN_3$ in einem Gemisch aus Wasser und Schwefelsäure umgesetzt wird.[37] Ein Upscaling der Reaktion zum *tert*Butylazid bis zu mehreren 100 g ist laut Literatur problemlos und sicherheitstechnisch möglich. Dennoch sollte bei dieser Syntheseroute stets bedacht werden, dass im Reaktionsgemisch $HN_3$ gebildet wird, welches unbedingt großzügig verdünnt gehandhabt werden sollte.

1.1 Synthese von *tert*Butylazid (nicht Bestandteil der Erfindung)

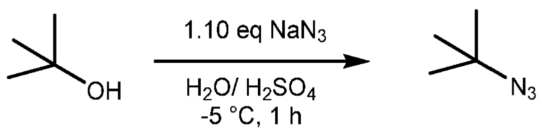


75 mL $H_2O$ und 50 mL konzentrierte $H_2SO_4$ wurden bei -5 °C vorgelegt. Mithilfe eines Feststoffdosierers wurde $NaN_3$ (9.80 g, 151 mmol, 1.10 eq) langsam zugegeben. Die farblose Suspension wurde für 15 min gerührt und anschließend auf 0 °C erwärmt. *t*BuOH (10.2 g, 137 mmol, 1.00 eq) wurde über einen Tropftrichter langsam zugetropft. Dabei löste sich der im Reaktionsgemisch vorliegende Feststoff langsam auf. Die Reaktionslösung wurde für 16 h bei RT gerührt und anschließend in einen Scheidetrichter überführt um eine Trennung der Phasen zu erreichen. Die wässrige Phase wurde abgetrennt und umgehend mit NaOH (2 m) neutralisiert. Die organische Phase wurde zweimal mit je 20 mL NaOH (2 m) gewaschen, anschließend über $Na_2SO_4$ getrocknet. Das gewünschte Produkt wurde umkondensiert und konnte mit einer Ausbeute von 69% (9.40 g, 94.8 mmol) in Form einer farblosen Flüssigkeit erhalten werden.

Beispiel 2: Synthese von H(dbt) und Li(dbt)

2.1 Synthese von Li(dbt) - Methode 1(nicht Bestandteil der Erfindung)

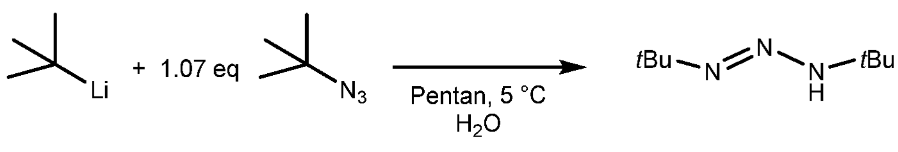

*t*Bu$N_3$ (10.17 g, 102.6 mmol, 1.00 eq) wurde in 100 mL Pentan vorgelegt und auf 5 °C gekühlt. Innerhalb einer Stunde wurde eine Lösung von *t*BuLi in Pentan (60 mL, 1.83 m, 110 mmol, 1.07 eq) zugetropft. Der Tropftrichter wurde zweimal mit 10 mL Pentan gespült. Das Reaktionsgemisch wurde langsam erwärmt und für 1 h bei RT gerührt. Zu der leicht gelben Suspension wurden 180 mL $H_2O$ zugeben, wodurch sich das Gemisch entfärbte. Die wässrige Phase wurde abgetrennt und die organische ein weiteres Mal mit 180 mL $H_2O$ gewaschen. Die organische Phase wurde über $Na_2SO_4$ getrocknet, filtriert und anschließend wurden alle flüchtigen Bestandteile im FV entfernt. Das Rohprodukt wurde destillativ bei 60 mbar und 70 °C aufgereinigt. Die Ausbeute des gewünschten Produktes in Form einer farblosen, klaren Flüssigkeit betrug 32% (5.15 g, 32.7 mmol).

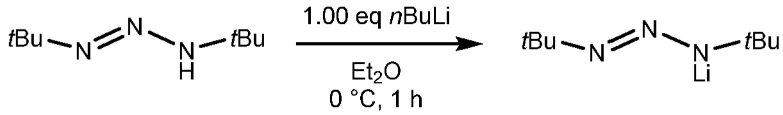


H(dbt) (700 mg, 4.45 mmol, 1.00 eq) wurde in 10 mL $Et_2O$ vorgelegt und auf 0 °C gekühlt. Eine Lösung von *n*BuLi in Hexan (1.8 mL, 2.5 m, 4.45 mmol, 1.00 eq) wurde langsam zugetropft. Die leicht gelbe Lösung wurde für 1 h bei RT gerührt, anschließend auf RT gebracht und für weitere 24 h gerührt. Das Lösungsmittel der klaren Lösung wurde im FV entfernt, wodurch ein leicht gelber Feststoff erhalten wurde. Das gewünschte Produkt wurde im FV getrocknet und konnte mit einer Ausbeute von 95% (687 mg, 4.21 mmol) isoliert werden.

2.2 Synthese von Li(dbt) - Methode 2 (optimierte Synthese) (nicht Bestandteil der Erfindung)

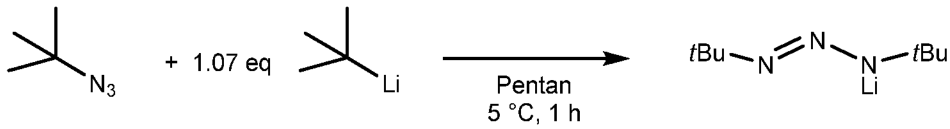


*t*Bu$N_3$ (1.27 g, 12.8 mmol, 1.00 eq) wurde in 10 mL Pentan vorgelegt und auf 5 °C gekühlt. Eine Lösung von *t*BuLi in Hexan (7.4 mL, 1.83 m, 13.7 mmol, 1.07 eq) wurde langsam zugetropft, wobei eine leichte Gelbfärbung des Reaktionsgemisches zu beobachten war. Das Reaktionsgemisch wurde 1 h bei 5 °C gerührt, anschließend langsam auf RT erwärmt und für 1 h gerührt. Die leicht trübe Lösung wurde über einen Spritzenfilter filtriert und das Lösungsmittel des leicht gelben Filtrats wurde im FV entfernt. Das gewünschte Produkt konnte mit einer Ausbeute von 83% (1.64 g, 10.0 mmol) erhalten werden.

Anhand durchgeführter analytischer Untersuchungen konnten keine Unterschiede zwischen den beiden Chargen Li(dbt) beobachtet werden. Bei der optimierten Synthese des Li(dbt) handelt es sich um eine einstufige Synthese ohne Nebenprodukte. Während zunächst vom Neutralligand ausgegangen wurde, führt die Optimierung sowohl zum Einsparen an Reagenzien als auch verschiedenster Arbeitsschritte, u.a. einer Destillation.

Beispiel 3: Synthese von K(dbt) (nicht Bestandteil der Erfindung)

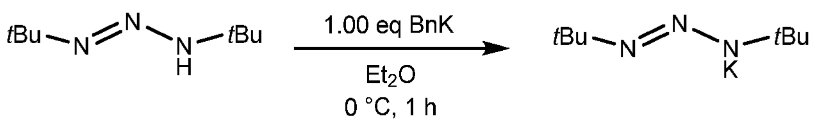


BnK (700 mg, 4.45 mmol, 1.00 eq) wurde in 10 mL $Et_2O$ aufgenommen und bei 0 °C tropfenweise mit H(dbt) (582 mg, 4.47 mmol, 1.00 eq) versetzt. Die farblose Suspension wurde auf RT erwärmt und für 16 h gerührt. Die leicht trübe Lösung wurde filtriert, das Lösungsmittel des farblosen Filtrats wurde im Unterdruck vollständig entfernt. Es konnten 85% (510 mg, 2.61 mmol) des gewünschten Produktes in Form eines farblosen Feststoffs erhalten werden.

Beispiel 4: Synthese von Li(mbt) (nicht Bestandteil der Erfindung)

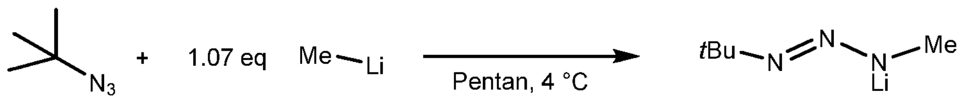


$tBuN_3$ (1.00 g, 10.7 mmol, 1.00 eq) wurde in 15 mL Pentan vorgelegt und auf 4 °C gekühlt. Eine Lösung von MeLi in $Et_2O$ (6.8 mL, 1.60 m, 10.7 mmol, 1.07 eq) wurde langsam tropfenweise zur Reaktionslösung zugegeben, wodurch ein farbloser Feststoff ausfiel. Das Reaktionsgemisch wurde auf RT erwärmt, der entstandene farblose Niederschlag abfiltriert und im FV für mehrere Stunden getrocknet. Das gewünschte Produkt konnte als farbloser Feststoff mit einer Ausbeute von 87% (1.13 g, 9.31 mmol) erhalten werden. Ein Upscaling der Reaktion ist möglich.

Beispiel 5: Synthese von H(mbt) (nicht Bestandteil der Erfindung)

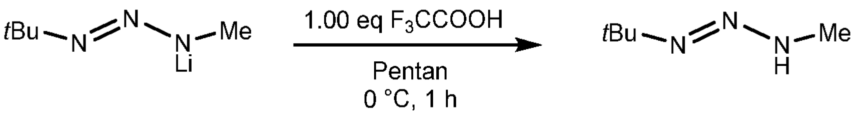


Li(mbt) (143 mg, 1.18 mmol, 1.00 eq) wurde in Pentan vorgelegt und auf 0 °C gekühlt. Unter Rühren wurde $F_3CCOOH$ (135 mg, 1.18 mmol, 1.00 eq) langsam zugetropft, wobei ein leichtes Aufschäumen der Reaktionslösung beobachtet werden konnte. Das eingesetzte Li(mbt) ging beim Erwärmen des Reaktionsgemischs auf RT in Lösung und die leicht trübe Suspension wurde für 16 h bei RT gerührt. Nachdem die Suspension über einen Spritzenfilter filtriert und das Pentan im FV entfernt wurde, konnte das gewünschte Produkt in Form einer farblosen Flüssigkeit erhalten werden.

Beispiel 6: Synthese von $[Al(dbt)_2(Me)]$

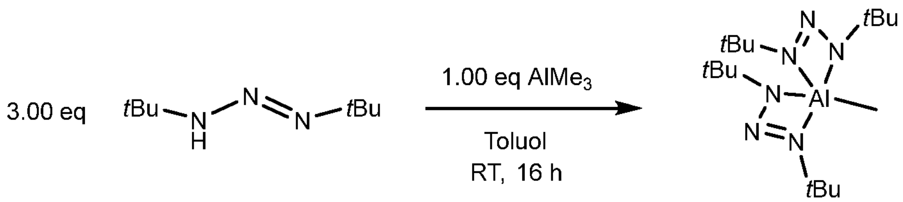


Eine toluolische Lösung von $AlMe_3$ (33 mg, 0.46 mmol, 1.00 eq) wurde vorgelegt und bei RT tropfenweise mit H(dbt) (300 mg, 1.91 mmol, 3.00 eq) versetzt. Dabei konnte umgehend eine Gasentwicklung beobachtet werden. Das farblose Reaktionsgemisch wurde für 72 h bei RT gerührt und anschließend über einen Spritzenfilter filtriert. Das Lösungsmittel des Filtrats wurde im FV entfernt, wodurch ein farbloses Öl erhalten wurde. Nach mehrmaligem Gefriertrocknen wurde das gewünschte Produkt mit einer Ausbeute von 87% (276 mg, 0.56 mmol) als farbloser Feststoff erhalten (Schmelzpunkt: 46 °C).

Thermogravimetrische Untersuchung von $[Al(dbt)_2(Me)]$

Das Rohprodukt wurde mittels thermogravimetrischer Analyse bis 700°C bei 10 K/min untersucht (FIG. 1). Die thermogravimetrische Analyse zeigt einen einstufigen Prozess mit einem Gesamtmasseabbau von circa 91,2%. Eine Gewichtsabnahme von 3% der Ausgangssubstanz wurde bei 127,2°C festgestellt.

Beispiel 7: Synthese von $[Ga(dbt)_2(H)]$

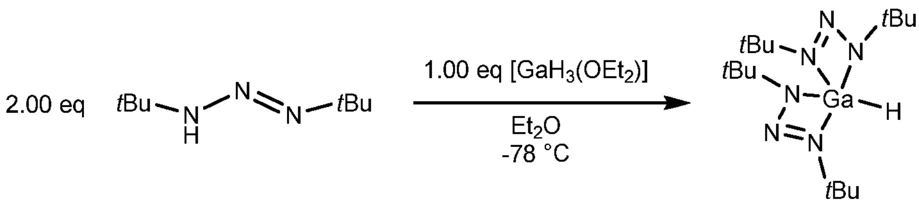


Eine auf -78 °C gekühlte Lösung von $GaCl_3$ (440 mg, 2.50 mmol, 1.00 eq) in 5 mL $Et_2O$ wurde zu einer Suspension aus LiH (260 mg, 32.8 mmol, 13.1 eq) in 5 mL $Et_2O$ getropft. Dabei fiel sofort ein leicht grauer Niederschlag aus. Das Reaktionsgemisch wurde für 2 h bei -78 °C, anschließend für 16 h bei RT gerührt. Die leicht graue Suspension wurde in einen auf vorgekühlten Kolben filtriert. Zu dem klaren Filtrat wurde bei -78 °C eine auf -78 °C gekühlte Lösung aus $GaCl_3$ (176 mg, 1.00 mmol, 0.40 eq) in 5 mL $Et_2O$ getropft. Die Suspension wurde unter Rühren bis auf 0 °C erwärmt und anschließend in einen auf -78 °C vorgekühlten Kolben filtriert. Das klare Filtrat wurde bei 0 °C zu einer Lösung aus H(dbt) (786 mg, 5.00 mmol, 2.00 eq) in 5 mL $Et_2O$ zugetropft. Dabei war umgehend eine Gasentwicklung zu beobachten. Die Suspension wurde langsam auf RT erwärmt und für 16 h gerührt. Das Gemisch wurde über einen Spritzenfilter filtriert und alle flüchtigen Bestandteile wurden im FV entfernt. Es blieb ein farbloser Feststoff zurück, welcher in 5 mL Hexan aufgenommen und erneut über einen Spritzenfilter filtriert wurde. Das Lösungsmittel des Filtrats wurde im FV entfernt, wodurch das Produkt mit einer Ausbeute 53% (508 mg, 1.33 mmol, Schmelzpunkt: 46 °C) erhalten wurde. Einkristalle für die Strukturanalyse konnten durch Sublimation im FV bei 60 °C erhalten werden.

Der entsprechende Dihydrido-Gallium-Komplex $[Ga(dbt)H_2]$ konnte nicht durch die 1:1-Umsetzung von H(dbt) und $[GaH_3(OEt_2)]$ erhalten werden.

Thermogravimetrische Untersuchung von $[Ga(dbt)_2(H)]$

Das Rohprodukt wurde mittels thermogravimetrischer Analyse bis 900°C bei 10 K/min untersucht (FIG. 2). Insgesamt wurden 86,6% der Ausgangssubstanz in die Gasphase überführt.

Beispiel 8: Synthese von $[In(dbt)Me_2]$

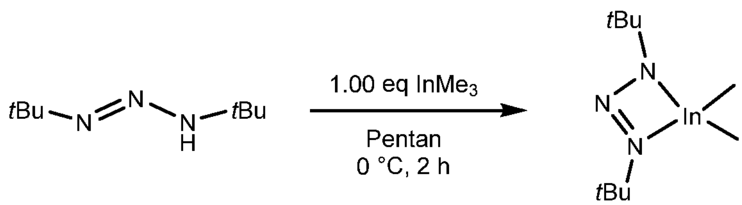


H(dbt) (250 mg, 1.59 mmol, 1.00 eq) wurde in 8 mL Pentan vorgelegt und auf 0 °C gekühlt. Eine toluolische Lösung von $InMe_3$ (254 mg, 1.59 mmol, 1.00 eq) wurde tropfenweise zugegeben, wobei eine leichte Gasentwicklung zu beobachten war. Das Reaktionsgemisch wurde für 1 h bei 0 °C und für 16 h bei RT gerührt. Das Lösungsmittel der klaren Lösung wurde im FV entfernt, wodurch das gewünschte Produkt als farblose Flüssigkeit isoliert werden konnte. Zur Aufreinigung kann das Produkt bei 40 °C im leichten Vakuum umkondensiert werden.

Die Reaktion kann in analoger Weise auch in Toluol durchgeführt werden, jedoch führt dies aufgrund der hohen Flüchtigkeit des Produktes zu Schwierigkeiten bei der Isolierung des Indium-Komplexes.

Beispiel 9: Synthese von $[In(dbt)_3]$

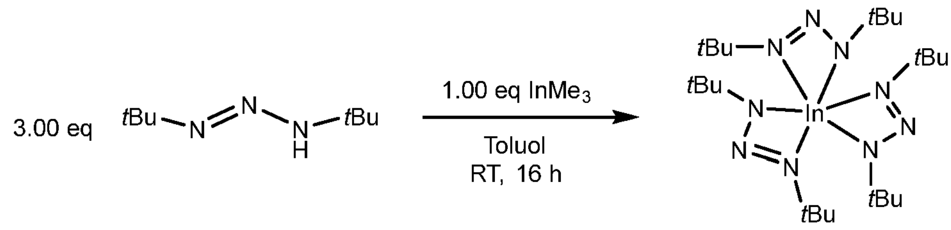


$InMe_3$ (150 mg, 0.94 mmol, 1.00 eq) wurde in 5 mL Toluol vorgelegt, auf 0 °C gekühlt und tropfenweise mit H(dbt) (444 mg, 2.83 mmol, 3.00 eq) versetzt. Das Reaktionsgemisch wurde langsam auf RT erwärmt und für 16 h gerührt. Das Lösungsmittel der klaren Lösung wurde im FV entfernt. Das gewünschte Produkt wurde in Form eines farblosen Feststoffs mit einer Ausbeute von 73% (403 mg, 0.69 mmol) erhalten und kann im FV bei 80 °C sublimiert werden.

Thermogravimetrische Untersuchung von $[In(dbt)_3]$

Das Rohprodukt wurde mittels thermogravimetrischer Analyse bis 900°C bei 10 K/min untersucht (FIG. 3). Die Analyse zeigt einen einstufigen Prozess mit einem Gesamtmasseabbau von circa 92,8%. Eine Gewichtsabnahme von 3% der Ausgangssubstanz wurde bei 196,7°C festgestellt.

Beispiel 10: Synthese von $[La(dbt)_3]$

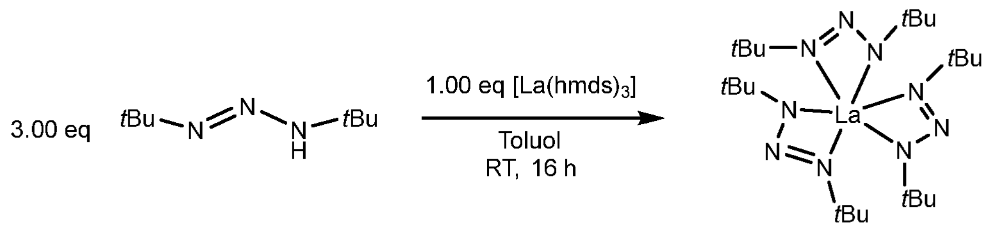


$[La(hmds)_3]$ (225 mg, 0.36 mmol, 1.00 eq) wurde in 10 mL Toluol vorgelegt und auf 0 °C gekühlt. Tropfenweise wurde H(dbt) (171 mg, 1.09 mmol, 3.00 eq) zu der farblosen Lösung gegeben. Das klare, farblose Reaktionsgemisch wurde auf RT erwärmt und für 16 h gerührt. Mithilfe einer $^1$H-NMR-Reaktionskontrolle wurde bestätigt, dass kein $[La(hmds)_3]$ mehr im Reaktionsgemisch vorhanden war. Alle flüchtigen Bestandteile der Reaktionslösung wurden im FV entfernt und der erhaltene farblose Feststoff wurde bei 60 °C im FV getrocknet. Das gewünschte Produkt wurde mit einer Ausbeute von 72% (158 mg, 0.26 mmol) erhalten.

Thermogravimetrische Untersuchung von $[La(dbt)_3]$

Das Rohprodukt wurde mittels thermogravimetrischer Analyse bis 800°C bei 10 K/min untersucht (FIG. 4). Die thermogravimetrische Analyse zeigt einen einstufigen Prozess mit einem Gesamtmasseabbau von circa 85%. Eine Gewichtsabnahme von 3% der Ausgangssubstanz wurde bei 85°C festgestellt. Im Bereich bis 100 °C lässt sich erkennen, dass noch Restspuren Hhmds im Produkt vorhanden sind. Ab einer Temperatur von 103 °C kommt es zu einem einsetzenden Schmelzprozess des Lanthan-Komplexes, wobei der maximale Masseabbau bei einer Temperatur von 250 °C erreicht ist. Ab einer Temperatur von ungefähr 400 °C lässt sich kein signifikanter Masseabbau mehr beobachten.

Beispiel 11: Synthese von $[Co(dbt)_2]$

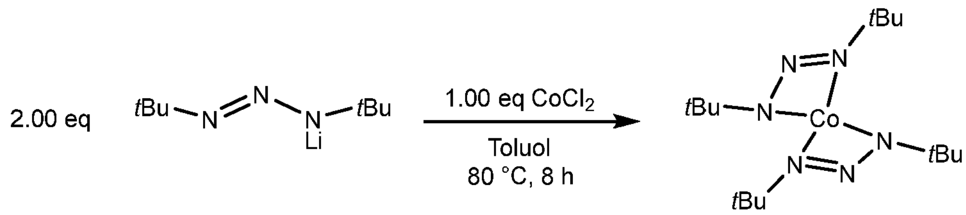


Li(dbt) (654 mg, 4.00 mmol, 2.00 eq) wurde zusammen mit $CoCl_2$ (260 mg, 2.00 mmol, 1.00 eq) vorgelegt und mit 15 mL Toluol versetzt. Das Reaktionsgemisch wurde für 8 h auf 80 °C erhitzt, wobei ein Farbwechsel von blau zu dunkelrot beobachtet werden konnte. Das Lösungsmittel des abgekühlten Reaktionsgemisches wurde im FV entfernt und das gewünschte Produkt wurde direkt aus dem Rückstand im FV bei 100 °C sublimiert. [Co(dbt)$_2$] wurde in Form eines dunkelroten, fast schwarzen Feststoffs mit einer Ausbeute von 53% (394 mg, 1.06 mmol) erhalten.

Thermogravimetrische Analyse und Rückstandsbestimmung von [Co(dbt)$_2$]

In der TGA-Kurve bis 900°C bei 10 K/min (FIG. 5) lässt sich ein einstufiger Masseverlust beobachten, bei dem der maximale Masseabbau pro Zeit bei einer Temperatur von 248 °C beobachtet werden kann. Bei einer Temperatur von 156 °C hat sich die Probe zu 3% abgebaut. Ab einer Temperatur von 91 °C lässt sich ein endothermer, sehr breiter Peak beobachten, welcher einem unregelmäßigen Schmelzvorgang zugeordnet werden kann. Der Gesamtmasseverlust liegt bei 97%, wobei der erhaltene Rückstand mittels XRPD näher untersucht wurde. Dabei zeigte sich, dass es sich bei dem Rückstand um elementares Cobalt handelt.

Beispiel 12: Synthese von [Co$_2$(mbt)$_4$]

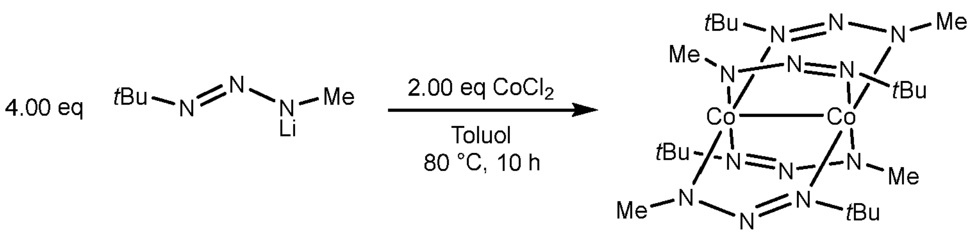


Li(mbt) (193 mg, 1.59 mmol, 4.00 eq) und $CoCl_2$ (103 mg, 0.79 mmol, 2.00 eq) wurden zusammen vorgelegt und bei 0 °C mit 10 mL Toluol versetzt. Beim Auftauen des Reaktionsgemisches konnte ein Farbwechsel von blau zu dunkelbraun beobachtet werden. Das Reaktionsgemisch wurde für 10 h auf 80 °C erhitzt und anschließend wurde das Lösungsmittel im FV bei RT entfernt. Aus dem dunkelbraunen Rückstand wurde das gewünschte Produkt im dynamischen Vakuum bei 85 °C sublimiert. Der dinukleare Cobalt-Komplex konnte als rotbrauner Feststoff mit einer Ausbeute von 51% (116 mg, 0.20 mmol) erhalten werden. Einkristalle für die Kristallstrukturanalyse konnten durch Sublimation im leichten Vakuumbei 100 °C erhalten werden. Aufgrund von Spinpaarung der beiden Cobalt-Kerne im [Co$_2$(mbt)$_4$], zeigt dieses im Gegensatz zu [Co(dbt)$_2$] diamagnetisches Verhalten.

Thermogravimetrische Analyse und Rückstandsbestimmung von [Co$_2$(mbt)$_4$]

Die TGA-Kurve bis 900°C bei 10 K/min (FIG. 6) des Cobalt-Komplexes zeigt einen einstufigen Verlauf, wobei der maximale Masseabbau pro Zeit bei einer Temperatur von 189 °C beobachtet werden kann. Der 3%- Abbau wurde bei einer Temperatur von 147 °C ermittelt. Ab einer Temperatur von 184 °C lässt sich ein einsetzender exothermer Prozess erkennen, welcher zum maximalen Masseabbau pro Zeit führt und einem Zersetzungsprozess zugeordnet werden kann. Der Gesamtmasseabbau liegt bei 82%, wobei der aus dieser Messung erhaltene Rückstand mittels XRPD untersucht wurde. Dieser konnte als elementares Cobalt identifiziert werden.

Beispiel 19: Synthese von [Cu$_2$(dbt)$_2$]

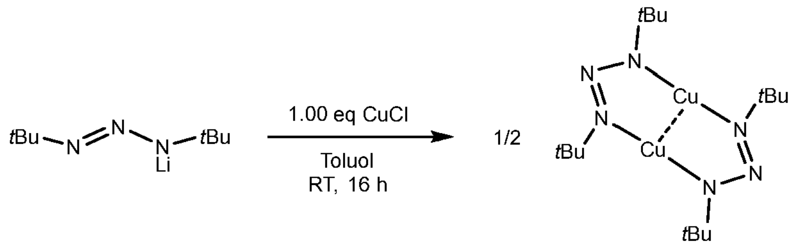


Li(dbt) (280 mg, 1.72 mmol, 1.00 eq) wurde zusammen mit CuCl (170 mg, 1.72 mmol, 1.00 eq) vorgelegt und mit 10 mL vorgekühlten Toluol versetzt. Das Reaktionsgemisch wurde für 16 h bei RT gerührt, wobei ein Farbwechsel von gelb zu braun beobachtet werden konnte. Die Suspension wurde über Celite® filtriert und anschließend das Lösungsmittel des Filtrats im FV entfernt. Das Rohprodukt wurde im dynamischen Vakuum bei 70 °C sublimativ aufgereinigt und konnte mit einer Ausbeute von 71% (268 mg, 0.61 mmol) als gelber Feststoff erhalten werden. Einkristalle für die Kristallstrukturanalyse wurden aus einer gesättigten Lösung von *n*-Hexan bei -21 °C erhalten.

Thermogravimetrische Untersuchung

Das Rohprodukt wurde mittels thermogravimetrischer Analyse bis 1000°C bei 10 K/min untersucht (FIG. 12). $[Cu_2(dbt)_2]$ zeigt einen einstufigen Masseabbau, wobei der 3%-Abbau bei 156 °C liegt. Der Gesamtmassenverlust liegt bei 96%, was auf die gute Sublimierbarkeit der Kupfer-Verbindung zurückzuführen ist. Ab einer Temperatur von circa 260 °C ist kein signifikanter Masseabbau mehr zu beobachten. Als Rückstand wurde elementares Kupfer im Tiegel beobachtet.

Beispiel 20: Synthese von $[Cu_4(mbt)_4]$

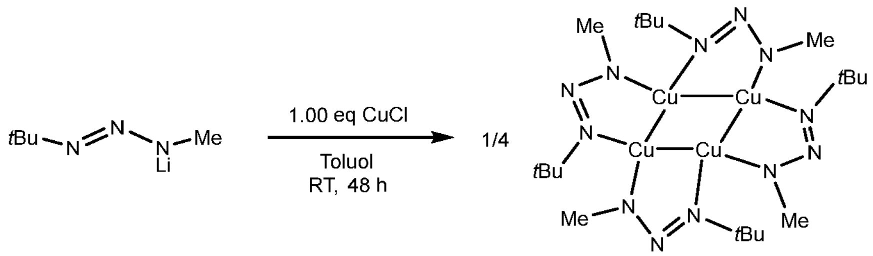


Li(mbt) (208 mg, 1.72 mmol, 1.00 eq) und CuCl (170 mg, 1.72 mmol, 1.00 eq) wurden vorgelegt und mit 10 mL vorgekühlten Toluol versetzt. Das farblose Reaktionsgemisch wurde für 48 h bei RT gerührt, wodurch eine Farbänderung zu leuchtend gelb beobachtet werden konnte. Die Suspension wurde über Celite® filtriert und das Lösungsmittel des Filtrats wurde im FV entfernt. Das leuchtend gelbe Rohprodukt wurde im dynamischen Vakuum bei 65 °C bis 75 °C sublimativ aufgereinigt und konnte mit einer Ausbeute von 82% (248 mg, 0.35 mmol) erhalten werden. Einkristalle für die Kristallstrukturanalyse konnten aus einer gesättigten Lösung von *n*-Hexan bei RT erhalten werden.

Thermogravimetrische Untersuchung

Das Rohprodukt wurde mittels thermogravimetrischer Analyse bis 900°C bei 10 K/min untersucht (FIG. 13). $[Cu_4(mbt)_4]$ zeigt einen einstufigen Abbau mit einem Gesamtmassenverlust von 79%. Der 3%-Abbau liegt bei 198 °C, während der maximale Masseabbau bei circa 250 °C bestimmt wurde. Ab einer Temperatur von 255 °C lässt sich kein signifikanter Masseverlust mehr beobachten. Die SDTA-Kurve zeigt verschiedene Schmelz- bzw. Phasenumwandlungsprozesse bis zur einsetzenden Zersetzung, welche bei einer Temperatur von 236 °C beginnt (nicht gezeigt). Der Rückstand aus der thermogravimetrischen Untersuchung wurde mittels XRPD untersucht und konnte als elementares Kupfer identifiziert werden.

Beispiel 21: Synthese von $[Ca(dbt)_2]$

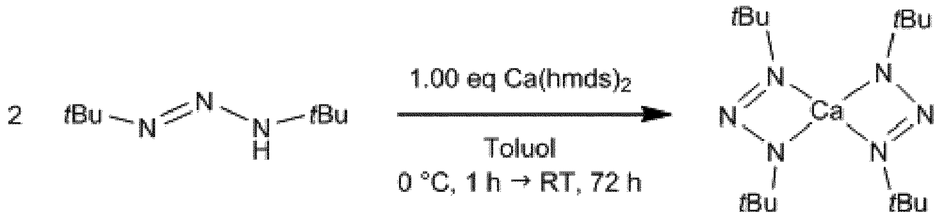


$Ca(hmds)_2$ (122 mg, 0.338 mmol, 1.00 eq) wurde in 10 mL Toluol vorgelegt und auf 0 °C gekühlt. Tropfenweise wurde Hdbt (106 mg, 0.684 mmol, 2.00 eq) hinzu gegeben. Das farblose Reaktionsgemisch wurde für 72 h bei RT gerührt, anschließend filtriert und das Filtrat bis zur Trockene eingedampft. Das Produkt konnte mit einer Ausbeute von 63% (116 mg, 0.22 mmol) als leicht gelber Feststoff erhalten werden.

**$^1$H-NMR ($C_6D_6$, 300 MHz, 300 K):** $\delta$/ ppm = 1.38 (s, C*Me*$_3$).

**$^{13}$C-NMR ($C_6D_6$, 75 MHz, 300 K):** $\delta$/ ppm = 31.1 (C*Me*$_3$), 56.2 (*C*Me$_3$).

**IR:** $\tilde{\upsilon}$ / cm$^{-1}$ = 2958 (m), 2860 (w), 1601 (w), 1473 (w), 1459 (w), 1384 (w), 1356 (m), 1295 (st), 1243 (m), 1194 (vst), 1026 (w), 989 (w), 806 (w), 748 (w), 614 (st), 472 (st).

**TGA:** (Ts = 25 °C, Tε = 800 °C, 10 °C / min), Stufen: 1

3%-Abbau: 115.3 °C, max. Abbau: 309.0 °C, Gesamtmassenabbau: 79.3%.

**SDTA:** $T_{D(Onset)}$: 194.4 °C, $T_{D(max.)}$: 263.7 °C.

**RPD:** Rückstand aus TGA-Analyse: $2\theta_{Lit.}$[62]($2\theta_{obs.}$) für $Ca_3N_2$: 32.779 (32.785), 35.598 (35.545), 36.191 (36.175), 37.279 (37.375), 38.611 (38.455), 44.599 (44.755), 50.979 (50.665), 60.458 (60.415), 66.228 (66.265).

Beispiel 31: Synthese von Cs(dbt)

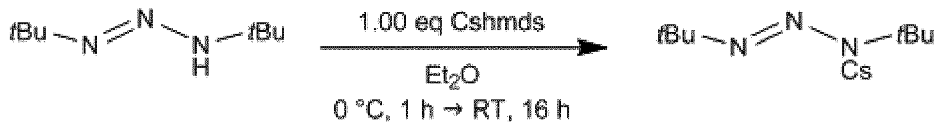


Cshmds wurde in 10 mL $Et_2O$ aufgenommen und bei 0 °C tropfenweise mit Hdbt versetzt. Die leicht trübe Lösung wurde auf RT erwärmt, für 16 h gerührt und über Celite® filtriert. Das Lösungsmittel des Filtrats wurde im FV entfernt. Ausbeute: 76%.

**$^1$H-NMR ($C_6D_6$/THF-$d_8$ (5/1), 300 MHz, 300 K):** $\delta$/ ppm = 1.35 (s, C*Me*$_3$).

**$^{13}$C-NMR ($C_6D_6$/THF-$d_8$ (5/1), 75 MHz, 300 K):** $\delta$/ ppm = 31.3 (C*Me*$_3$), 55.8 (*C*Me$_3$).

**Elementaranalyse:** für $C_8H_{18}CsN_3$

| | |
|---|---|
| berechnet: | C: 33.23%, H: 6.27%, N: 14.53%. |
| gefunden: | C: 32.60%, H: 6.07%, N: 14.64%. |

Beispiel 34: Synthese von [Ga(mbt)$Me_2$]

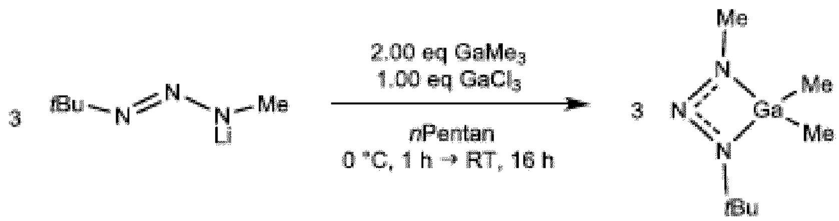


$GaMe_3$ (146 mg, 1.27 mmol, 2.00 eq) wurde in 5 mL *n*Pentan vorgelegt und bei 0 °C zu einer Lösung von $GaCl_3$ (112 mg, 0.64 mmol, 1.00 eq) in 10 mL *n*Pentan gegeben. Die farblose Lösung wurde auf RT erwärmt, für 16 h gerührt, erneut auf 0°C gekühlt und zu einer Lösung von [Li(mbt)] (231 mg, 1.91 mmol, 3.00 eq) in 10 mL *n*Pentan gegeben. Das Reaktionsgemisch wurde auf RT erwärmt und für 16 h gerührt. Das Lösungsmittel der Suspension wurde

im FV entfernt und das gewünschte Produkt aus dem Rückstand mittels Kondensation im FV isoliert. Das Produkt wurde in Form einer farblosen Flüssigkeit mit einer Ausbeute von 57% (233 mg, 1.09 mmol) erhalten.

**$^1$H-NMR ($C_6D_6$, 300 MHz, 300 K):** $\delta$ / ppm = 0.05 (s, 6 H, Ga*Me*$_2$), 1.17 (s, 18 H, C*Me*$_3$), 3.03 (s, 3 H, N*Me*).

**$^{13}$C-NMR ($C_6D_6$, 75 MHz, 300 K):** $\delta$ / ppm = -5.6 (Ga*Me*$_2$), 29.7 (C*Me*$_3$), 39.4 (N*Me*).

**IR:** $\tilde{\upsilon}$ / $cm^{-1}$ = 2969 (m), 2902 (w), 1524 (w), 1459 (w), 1435 (m), 1361 (vst), 1309 (m), 1271 (w), 1199 (vst), 1068 (m), 1026 (w), 955 (w), 930 (w), 866 (st), 787 (w), 737 (st), 688 (m), 646 (st), 572 (vst), 534 (vst), 481 (w), 460 (m), 438 (w).

**TGA:** (Ts = 25 °C, Tε = 450 °C, 10 °C / min), Stufen: 1
3%-Abbau: 63.2 °C, max. Abbau: 100.9 °C, Gesamtmasseabbau: 87.4%.

**SDTA:** $T_{D(Onset)}$: 81.6 °C, $T_{D(max)}$: 100.3 °C.
Fig. 21 zeigt eine graphische Darstellung der TGA- und SDTA-Messungen.

Beispiel 35: Synthese von [Ga(dbt)$Me_2$]

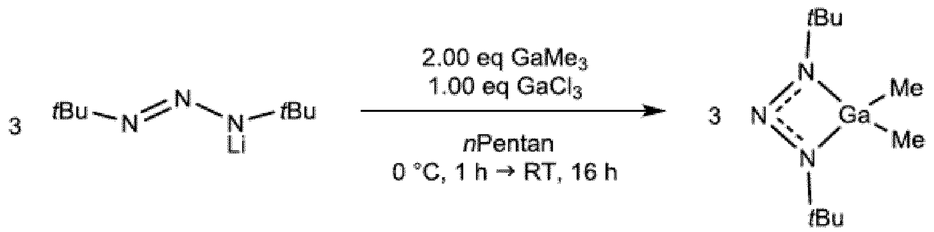


$GaMe_3$ (181 mg, 1.58 mmol, 2.00 eq) wurde in 10 mL *n*Pentan vorgelegt und bei 0 °C zu einer Lösung von $GaCl_3$ (139 mg, 0.79 mmol, 1.00 eq) in 10 mL *n*Pentan gegeben. Das Reaktionsgemisch wurde auf RT erwärmt und 16 h gerührt. Bei 0 °C wurde eine Lösung von [Li(dbt)] (387 mg, 2.37 mmol, 3.00 eq) zugetropft, wobei umgehend das Ausfallen eines farblosen Feststoffs beobachtet wurde. Die Suspension wurde langsam auf RT erwärmt und für 16 h gerührt. Das Lösungsmittel wurde im Feinvakuum (FV) entfernt und das Produkt in Form einer farblosen Flüssigkeit aus dem Rückstand im FV kondensiert. Das Produkt wurde mit einer Ausbeute von 43% (261 mg, 1.02 mmol) erhalten.

**$^1$H-NMR ($C_6D_6$, 300 MHz, 300 K):** $\delta$/ ppm = 0.10 (s, 6 H, Ga*Me*$_2$), 1.17 (s, 18 H, C*Me*$_3$).

**$^{13}$C-NMR ($C_6D_6$, 75 MHz, 300 K):** $\delta$ / ppm = -5.2 (Ga*Me*$_2$), 29.7 (C*Me*$_3$), 55.8 (N*Me*).

**IR:** $\tilde{\upsilon}$ / $cm^{-1}$ = 2967 (st), 2870 (w), 1459 (w), 1387 (w), 1361 (m), 1295 (vst), 1217 (vst), 1029 (w), 924 (w), 767 (m), 699 (m), 630 (m), 583 (st), 545 (st), 484 (m).

**TGA:** ($T_S$ = 25 °C, $T_E$ = 450 °C, 10 °C / min), Stufen: 1
3%-Abbau: 64.2 °C, max. Abbau: 116.0 °C, Gesamtmasseabbau: 89.9%.

**SDTA:** $T_{D(Onset)}$: 79.5 °C, $T_{D(max\cdot)}$: 115.7 °C.
Fig. 22 zeigt eine graphische Darstellung der TGA- und SDTA-Messungen.

Beispiel 36: Synthese von [Al(mbt)$_3$]

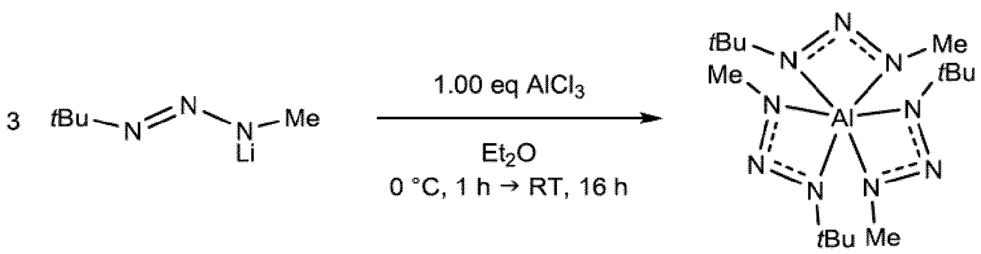


Eine Lösung von $AlCl_3$ (131 mg, 0.98 mmol, 1.00 eq) in 10 mL $Et_2O$ wurde bei 0°C zu einer Lösung von [Li(mbt)] (357 mg, 2.95 mmol, 3.00 eq) in 10 mL $Et_2O$ getropft. Das farblose Reaktionsgemisch wurde langsam auf RT erwärmt, für 16 h gerührt und anschließend filtriert. Der Rückstand wurde mit 10 mL $Et_2O$ extrahiert und das Filtrat im FV bis zur Trockene eingedampft. Der blass gelbe Feststoff wurde im Feinvakuum bei 45 °C sublimiert und das Produkt mit einer Ausbeute von 21% (76 mg, 0.21 mmol) erhalten. **$^1$H-NMR ($C_6D_6$, 300 MHz, 300 K):** $\delta$ / ppm = 1.31 (s, 27 H, C*Me*$_3$),

3.16 (s, 9 H, N*Me*). **$^{13}$C-NMR ($C_6D_6$, 75 MHz, 300 K):** $\delta$ / ppm = 30.3 (C*Me*$_3$), 37.5 (N*Me*), 55.8 (*C*Me$_3$). **$^{27}$Al-NMR ($C_6D_6$, 130 MHz, 300 K):** $\delta$ / ppm = 28.1.

**IR:** $\tilde{\upsilon}$ / cm$^{-1}$ = 2968 (m), 2926 (m), 2896 (m), 2802 (w), 1473 (w), 1457 (w), 1415 (w), 1387 (w), 1358 (m), 1300 (vst), 1263 (vst), 1229 (st), 1199 (st), 1106 (w), 1025 (w), 954 (w), 796 (w), 625 (m), 572 (w), 524 (vst), 435 (w).

**Elementaranalyse:** für $C_{15}H_{36}AlN_9$

berechnet: C: 48.76%, H: 9.82%, N: 34.12%. gefunden: C: 47.95%, H: 9.63%, N: 33.55%.

**TGA:** (Ts = 25 °C, Tε = 700 °C, 10 °C / min), Stufen: 1

3%-Abbau: 124.6 °C, max. Abbau: 216.1 °C, Gesamtmasseabbau: 96.4%.

**SDTA:** $T_{M(Onset)}$: 45.6 °C, $T_{M(max)}$: 49.7 °C, To(onset): 205.1 °C, $T_{D(max.)}$: 218.8 °C.

Fig. 23 zeigt eine graphische Darstellung der TGA- und SDTA-Messungen.

Beispiel 37: Synthese von [Al(dbt)$_3$]

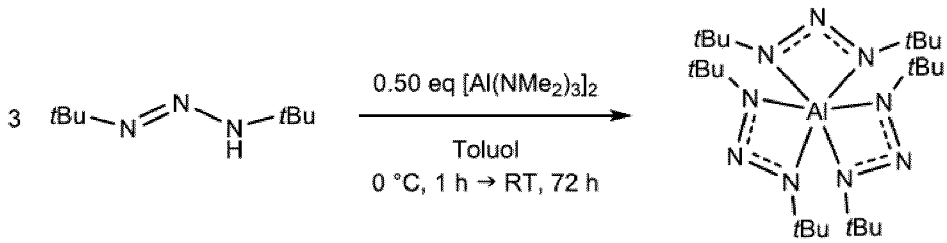


$[Al(NMe_2)_3]_2$ (154 mg, 0.97 mmol, 0.50 eq) wurde in 10 mL Toluol vorgelegt, auf 0 °C gekühlt und tropfenweise mit H(dbt) (458 mg, 2.91 mmol, 3.00 eq) versetzt. Die Reaktionslösung wurde für 1 h bei 0 °C gerührt und anschließend auf RT erwärmt. Dabei war eine Gasentwicklung zu beobachten. Nachdem die gelbe Lösung für 16 h bei RT gerührt wurde, konnte durch Entfernen des Lösungsmittels im FV ein schwach gelber Feststoff erhalten werden. Das Rohprodukt wurde bei 55 °C im FV sublimativ aufgereinigt und als farbloser Feststoff mit einer Ausbeute von 51% (244 mg, 0.49 mmol) isoliert.

**$^{1}$H-NMR ($C_6D_6$, 300 MHz, 300 K):** $\delta$ / ppm = 1.38 (s, C*Me*$_3$).

**$^{13}$C-NMR ($C_6D_6$, 75 MHz, 300 K):** $\delta$ / ppm = 31.2 (C*Me*$_3$), 57.2 (*C*Me$_3$).

**$^{27}$Al-NMR ($C_6D_6$, 130 MHz, 300 K):** $\delta$ / ppm = 24.6.

**IR:** $\tilde{\upsilon}$ / cm$^{-1}$ = 2972 (m), 2929 (s), 2870 (s), 2812 (s), 2767 (s), 1474 (s), 1387 (s), 1360 (m), 1302 (vst), 1257 (st), 1201 (vst), 1160 (st), 1069 (s), 1034 (s), 977 (m), 899 (s), 845 (s), 767 (s), 627 (m), 570 (m), 543 (vst), 438 (s).

**Elementaranalyse:** für $C_{24}H_{54}AlN_9$ berechnet: C: 58.15%, H: 10.98%, N: 25.43%. gefunden: C: 58.25%, H: 10.13%, N: 24.32%.

**TGA:** (Ts = 25 °C, Tε = 700 °C, 10 °C / min), Stufen: 1

3%-Abbau: 126.8 °C, max. Abbau: 172.6, 302.0 °C, Gesamtmasseabbau: 95.3%.

**SDTA:** $T_{D(Onset)}$: 270.0 °C, $T_{D(max.)}$: 302.7 °C. Fig. 24 zeigt eine graphische Darstellung der TGA- und SDTA-Messungen.

Literaturverzeichnis

[1] J. C. Brand, B. P. Roberts, J. Chem. Soc. Chem. Comm. 1981, 748-749.
[2] R. H. J. Smith, C. J. Michejda, Synthesis 1983, 476-477.
[3] R. H. J. Smith, B. D. Wladowski, A. F. Mehl, M. J. Cleveland, E. A. Rudrow, G. N. Chmurny, C. J. Michejda, J. Org. Chem. 1989, 54, 1036-1042.
[4] D. H. Sieh, D. J. Wilbur, C. J. Michejda, J. Am. Chem. Soc. 1980, 3883-3887.
[5] Soussi, K., Mishra, S, Jeanneau, E, Millet, J.-M., Daniele, S., Dalton Transactions 2017, 46, 38, 13055 bis 13064
[6] C. Lego, B. Neumüller, Z. Anorg. Allg. Chem. 2011, 637, 1784-1789.
[7] P. Gantzel, P. J. Walsh, Inorg. Chem. 1998, 37, 3450-3451.
[8] D. Kalden, S. Krieck, H. Görls, M. Westerhausen, Dalt. Trans. 2015, 44, 8089-8099.
[9] H. S. Lee, S.-O. Hauber, D. Vindus, M. Niemeyer, Inorg. Chem. 2008, 47, 4401-4412.
[10] S. L. Hyui, M. Niemeyer, Inorg. Chem. 2006, 45, 6126-6128.
[11] F. E. Brinckman, H. S. Haiss, R. A. Robb, Inorg. Chem. 1965, 4, 936-942.

[12] J. T. Leman, A. R. Barron, J. W. Ziller, R. M. Kren, Polyhedron 1989, 8, 1909-1912.
[13] J. T. Leman, H. A. Roman, A. R. Barron, J. Chem. Soc. Dalt. Trans. 1992, 1, 2183.
[14] M. L. Cole, A. J. Davies, C. Jones, P. C. Junk, A. I. McKay, A. Stasch, Z. Anorg. Allg. Chem. 2015, 641, 2233-2244.
[15] S. G. Alexander, M. L. Cole, C. M. Forsyth, S. K. Furfari, K. Konstas, Dalt. Trans. 2009, 2326-2336.
[16] J. T. Leman, J. Braddock-Wilking, A. J. Coolong, A. R. Barron, Inorg. Chem. 1993, 32, 4324-4336.
[17] I. A. Guzei, L. M. Liable-Sands, A. L. Rheingold, C. H. Winter, Polyhedron 1997, 16, 4017-4022.
[18] V. C. Gibson, D. F. Reardon, A. K. Tomov, 2004, WO 2004063233.
[19] E. Hartmann, J. Strähle, J. Inorg. Gen. Chem. 1990, 583, 31-40.
[20] I. D. Brown, J. D. Dunitz, Acta Cryst. 1961, 14, 480-485.
[21] L. R. Falvello, E. P. Urriolabettia, U. Mukhopadhyay, D. Ray, Acta Cryst. Comm. 1999, C55, 170-172.
[22] H. S. Lee, M. Niemeyer, Inorg. Chim. Acta 2011, 374, 163-170.
[23] A. L. Johnson, A. M. Willcocks, S. P. Richards, Inorg. Chem. 2009, 48, 8613-8622.
[24] E. Pfeiffer, M. W. Kokkes, K. Vrieze, Transit. Met. Chem. 1979, 4, 389-393.
[25] M. Venter, I. Haiduc, L. David, O. Cozar, J. Mol. Struct. 1997, 408-409, 483-486.
[26] F. A. Cotton, X. Feng, Inorg. Chem. 1989, 28, 1180-1183.
[27] J. G. M. van der Linden, A. H. Dix, E. Pfeiffer, Inorg. Chim. Acta 1980, 39, 271-274.
[28] S. F. Colson, S. D. Robinson, Inorg. Chim. Acta Lett. 1988, 149, 13-14.
[29] S. F. Colson, S. D. Robinson, M. Motevalli, M. B. Hursthouse, Polyhedron 1988, 7, 1919-1924.
[30] J. A. Cabeza, J. M. Fernhdez-Colinas, Coord. Chem. Rev. 1993, 126, 319-336.
[31] C. J. Weiss, T. J. Marks, Dalt. Trans. 2010, 39, 6576.
[32] B. Liu, D. Cui, Dalt. Trans. 2009, 550-556.
[33] P. J. Eulgem, A. Klein, N. Maggiarosa, D. Naumann, R. W. H. Pohl, Chem. Eur. J. 2008, 14, 3727-3736.
[34] S. G. Alvarez, M. T. Alvarez, Synthesis 1997, 4, 413-414.
[35] P. Margaretha, S. Solar, O. E. Polansky, Angew. Chemie - Int. Ed. 1971, 10, 412-413.
[36] P. Margaretha, S. Solar, O. E. Polansky, Angew. Chemie 1971, 11, 410.
[37] J. C. Bottaro, P. E. Penwell, R. J. Schmitt, Synth. Commun. 1997, 27, 1465-1467.

## Patentansprüche

1. Verwendung eines Metallkomplexes der Formel (2):

$$M[(L^1)_aX_d] \qquad (2)$$

wobei

$L^1$ die Formel $R^1$-$N_3$-$R^2$ aufweist, wobei $R^1$ und $R^2$ Alkylreste mit 1 bis 12 C-Atomen sind,
X ausgewählt ist aus H, Halogen, CO und Alkyl mit 1 bis 6 C-Atomen,
a = 2 oder 3 ist,
d = 0 oder 1 ist, und
M ausgewählt ist aus In, Co, Cu, Al, Ga, Tl und La zum Abscheiden des Metalls oder einer Verbindung des Metalls aus der Gasphase.

2. Verwendung gemäß Anspruch 1, wobei die Alkylreste $R^1$ und $R^2$ 1 bis 6 C-Atome aufweisen.

3. Verwendung gemäß mindestens einem der vorhergehenden Ansprüche, wobei Alkylreste $R^1$ und $R^2$ 1 bis 4 C-Atome aufweisen.

4. Verwendung gemäß mindestens einem der vorhergehenden Ansprüche, wobei alle oder ein Teil der Reste $R^1$ und/oder $R^2$ verzweigte Alkylreste sind.

5. Verwendung gemäß mindestens einem der vorhergehenden Ansprüche, wobei der Metallkomplex homoleptisch ist und ausschließlich Liganden **L** der Formel $R^1$-$N_3$-$R^2$ aufweist.

6. Verwendung gemäß mindestens einem der vorhergehenden Ansprüche, wobei der Metallkomplex heteroleptisch ist.

7. Verwendung gemäß mindestens einem der vorhergehenden Ansprüche, wobei die Reste $R^1$ und $R^2$ gleich sind.

**8.** Verwendung gemäß mindestens einem der vorhergehenden Ansprüche, wobei die Reste $R^1$ und $R^2$ ausgewählt aus Methyl, Ethyl, Propyl, Isopropyl, *tert*-Butyl und n-Propyl sind.

**9.** Verwendung gemäß mindestens einem der vorhergehenden Ansprüche, wobei alle oder ein Teil der Reste $R^1$ und/oder $R^2$ *tert*-Butyl sind.

**10.** Verwendung mindestens einem der vorhergehenden Ansprüche, wobei der Metallkomplex mindestens einen Liganden **L** aufweist, der *tert*-Butyl-$N_3$-*tert*-Butyl oder *tert*-Butyl-$N_3$-Methyl ist.

**11.** Verwendung gemäß Anspruch 10 wobei alle Liganden **L** des Metallkomplexes *tert*-Butyl-$N_3$-*tert*-Butyl oder *tert*-Butyl-$N_3$-Methyl sind.

**12.** Verwendung gemäß mindestens einem der vorhergehenden Ansprüche, wobei der Metallkomplex sublimierbar oder verdampfbar ist, ohne dass eine Zersetzung erfolgt.

**13.** Verwendung gemäß mindestens einem der vorhergehenden Ansprüche, wobei das Molekulargewicht des Metallkomplexes kleiner ist als kleiner als 600 g/Mol.

**14.** Verwendung gemäß mindestens einem der vorhergehenden Ansprüche, wobei der Metallkomplex bei 100°C thermisch stabil ist und/oder in einem Temperaturbereich von 100°C bis 400°C zerfällt, wobei die Temperatur bei Normaldruck oder bei reduziertem Druck im Bereich von $10^{-3}$ bis 900 mbar ermittelt wird.

**15.** Verwendung gemäß mindestens einem der vorhergehenden Ansprüche, wobei das Abscheiden des Metalls oder der Verbindung des Metalls aus der Gasphase bei reduziertem Druck im Bereich von $10^{-3}$ bis 900 mbar, bevorzugt im Bereich von $10^{-2}$ bis 1 mbar, und insbesondere $10^{-2}$ mbar erfolgt.

**16.** Verfahren zur Herstellung von beschichteten Substraten, umfassend die Schritte:

(a) Bereitstellen eines Metallkomplexes gemäß mindestens einem der vorhergehenden Ansprüche, und
(b) Abscheiden des Metalls oder einer Verbindung des Metalls auf der Oberfläche des Substrats durch metallorganische Gasphasenabscheidung.

**17.** Verfahren oder Verwendung gemäß mindestens einem der vorhergehenden Ansprüche, wobei das Verfahren eine metallorganische chemische Gasphasenabscheidung (MOCVD) ist.

**18.** Verfahren oder Verwendung gemäß mindestens einem der vorhergehenden Ansprüche, wobei das Verfahren eine metallorganische Gasphasenepitaxie (MOVPE) ist.

**19.** Verfahren oder Verwendung gemäß mindestens einem der vorhergehenden Ansprüche, wobei die Verbindung des Metalls aus Halbleiterverbindungen, Legierungen, Nitriden, Phosphiden, Arseniden und Siliziden ausgewählt ist.

**20.** Verfahren oder Verwendung gemäß mindestens einem der vorhergehenden Ansprüche, wobei der Metallkomplex sublimiert oder verdampft wird, ohne dass eine Zersetzung erfolgt.

**21.** Verfahren oder Verwendung gemäß mindestens einem der vorhergehenden Ansprüche, wobei sich der Metallkomplex in der Gasphase zersetzt bei einer Temperatur, die nicht mehr als 100°C oberhalb der Sublimationstemperatur oder Verdampfungstemperatur liegt.

## Claims

**1.** Use of a metal complex of the Formula (2):

$$M[(L^1)_aX]_d \qquad (2)$$

whereby

$L^1$ has the formula $R^1$-$N_3$-$R^2$, where $R^1$ and $R^2$ are alkyl radicals having 1 to 12 carbon atoms,
X is selected from H, halogen, CO and alkyl with 1 to 6 C atoms,
a = 2 or 3,
d = 0 or 1, and
M is selected from In, Co, Cu, Al, Ga, Tl and La for depositing the metal or a compound of the metal from the gas phase.

**2.** Use according to claim 1, wherein the alkyl radicals $R^1$ and $R^2$ have 1 to 6 carbon atoms.

**3.** Use according to at least one of the preceding claims, wherein alkyl radicals $R^1$ and $R^2$ have 1 to 4 carbon atoms.

**4.** Use according to at least one of the preceding claims, wherein all or part of the radicals $R^1$ and/or $R^2$ are branched alkyl radicals.

**5.** Use according to at least one of the preceding claims, wherein the metal complex is homoleptic and comprises exclusively ligands **L** of the formula $R^1$-$N_3$-$R^2$.

**6.** Use according to at least one of the preceding claims, wherein the metal complex is heteroleptic.

**7.** Use according to at least one of the preceding claims, wherein the radicals $R^1$ and $R^2$ are identical.

**8.** Use according to at least one of the preceding claims, wherein the radicals $R^1$ and $R^2$ are selected from methyl, ethyl, propyl, isopropyl, *tert-butyl* and n-propyl.

**9.** Use according to at least one of the preceding claims, wherein all or part of the radicals $R^1$ and/or $R^2$ are *tert-butyl.*

**10.** Use of at least one of the preceding claims, wherein the metal complex comprises at least one ligand **L** which is tert-butyl-$N_3$-*tert-butyl* or tert-butyl-$N_3$ -methyl.

**11.** Use according to claim 10, wherein all ligands **L** of the metal complex are tert-butyl-$N_3$ -*tert-butyl* or tert-butyl-$N_3$-methyl.

**12.** Use according to at least one of the preceding claims, wherein the metal complex is sublimable or vaporizable without decomposition taking place.

**13.** Use according to at least one of the preceding claims, wherein the molecular weight of the metal complex is less than less than 600 g/mol.

**14.** Use according to at least one of the preceding claims, wherein the metal complex is thermally stable at 100°C and/or decomposes in a temperature range from 100°C to 400°C, wherein the temperature is determined at normal pressure or at reduced pressure in the range from $10^{-3}$ to 900 mbar.

**15.** Use according to at least one of the preceding claims, wherein the separation of the metal or the compound of the metal from the gas phase takes place at reduced pressure in the range from $10^{-3}$ to 900 mbar, preferably in the range from $10^{-2}$ to 1 mbar, and in particular $10^{-2}$ mbar.

**16.** A process for the production of coated substrates comprising the steps of:

(a) Providing a metal complex according to at least one of the preceding claims, and
(b) Deposition of the metal or a compound of the metal on the surface of the substrate by organometallic vapor deposition.

**17.** The method or use according to at least one of the preceding claims, wherein the method is metal organic chemical vapor deposition (MOCVD).

**18.** A method or use according to at least one of the preceding claims, wherein the method is organometallic vapor phase epitaxy (MOVPE).

19. The method or use according to at least one of the preceding claims, wherein the compound of the metal is selected from semiconductor compounds, alloys, nitrides, phosphides, arsenides and silicides.

20. Process or use according to at least one of the preceding claims, wherein the metal complex is sublimated or vaporized without decomposition taking place.

21. The method or use according to at least one of the preceding claims, wherein the metal complex decomposes in the gas phase at a temperature not more than 100°C above the sublimation temperature or evaporation temperature.

**Revendications**

1. Utilisation d'un complexe métallique de la Formule (2) :

$$M[(L^1)_a X_d] \qquad (2)$$

où

$L^1$ présente la formule $R^1$-$N_3$-$R^2$, où $R^1$ et $R^2$ sont des radicaux alkyle comportant de 1 à 12 atomes de carbone,
X est choisi parmi H, un halogène, CO et un groupe alkyle ayant de 1 à 6 atomes de carbone,
a = 2 ou 3,
d = 0 ou 1, et
M est choisi parmi In, Co, Cu, Al, Ga, Tl et La pour déposer le métal ou un composé du métal à partir de la phase gazeuse.

2. Utilisation selon la revendication 1, dans laquelle les radicaux alkyle $R^1$ et $R^2$ présentent 1 à 6 atomes de carbone.

3. Utilisation selon au moins l'une des revendications précédentes, dans laquelle les radicaux alkyle $R^1$ et $R^2$ présentent 1 à 4 atomes de carbone.

4. Utilisation selon au moins l'une des revendications précédentes, dans laquelle tout ou partie des radicaux $R^1$ et/ou $R^2$ sont des radicaux alkyle ramifiés.

5. Utilisation selon au moins l'une des revendications précédentes, dans laquelle le complexe métallique est homoleptique et comporte exclusivement des ligands **L** de formule $R^1$-$N_3$-$R^2$.

6. Utilisation selon au moins l'une des revendications précédentes, dans laquelle le complexe métallique est hétéroleptique.

7. Utilisation selon au moins l'une des revendications précédentes, dans laquelle les radicaux $R^1$ et $R^2$ sont identiques.

8. Utilisation selon au moins l'une des revendications précédentes, dans laquelle les radicaux $R^1$ et $R^2$ sont choisis parmi les radicaux méthyle, éthyle, propyle, isopropyle, *tert-butyle* et n-propyle.

9. Utilisation selon au moins l'une des revendications précédentes, dans laquelle tout ou partie des radicaux $R^1$ et/ou $R^2$ sont des radicaux *tert-butyle.*

10. Utilisation d'au moins l'une des revendications précédentes, dans laquelle le complexe métallique comprend au moins un ligand **L** qui est le *tert*-butyle-$N_3$-*tert*-butyle ou le *tert* -butyl-$N_3$-méthyle.

11. Utilisation selon la revendication 10, dans laquelle tous les ligands **L** du complexe métallique sont le *tert*-butyle-$N_3$-*tert*-butyle ou le *tert*-butyl-$N_3$-méthyle.

12. Utilisation selon au moins l'une des revendications précédentes, dans laquelle le complexe métallique peut être sublimé ou évaporé sans se décomposer.

13. Utilisation selon au moins l'une des revendications précédentes, dans laquelle le poids moléculaire du complexe

métallique est inférieur à moins de 600 g/mol.

**14.** Utilisation selon au moins l'une des revendications précédentes, dans laquelle le complexe métallique est thermiquement stable à 100°C et/ou se décompose dans une plage de températures de 100°C à 400°C, la température étant déterminée à pression normale ou à pression réduite dans la plage de $10^{-3}$ à 900 mbar.

**15.** Utilisation selon au moins l'une des revendications précédentes, dans laquelle le dépôt du métal ou du composé du métal à partir de la phase gazeuse s'effectue à une pression réduite comprise entre $10^{-3}$ et 900 mbar, de préférence entre $10^{-2}$ et 1 mbar, et en particulier $10^{-2}$ mbar.

**16.** Procédé de fabrication de substrats revêtus, comprenant les étapes consistant à :

(a) fournir un complexe métallique selon au moins l'une des revendications précédentes, et
(b) Dépôt du métal ou d'un composé du métal sur la surface du substrat par dépôt organométallique en phase vapeur.

**17.** Procédé ou utilisation selon au moins l'une des revendications précédentes, dans lequel le procédé est un dépôt chimique en phase vapeur organométallique (MOCVD).

**18.** Procédé ou utilisation selon au moins l'une des revendications précédentes, dans lequel le procédé est une épitaxie en phase gazeuse organométallique (MOVPE).

**19.** Procédé ou utilisation selon au moins l'une des revendications précédentes, dans lequel le composé du métal est choisi parmi les composés semi-conducteurs, les alliages, les nitrures, les phosphures, les arséniures et les siliciures.

**20.** Procédé ou utilisation selon au moins l'une des revendications précédentes, dans lequel le complexe métallique est sublimé ou évaporé sans qu'il y ait décomposition.

**21.** Procédé ou utilisation selon au moins l'une des revendications précédentes, dans lequel le complexe métallique se décompose en phase gazeuse à une température qui n'est pas supérieure de plus de 100°C à la température de sublimation ou à la température d'évaporation.

FIG. 1

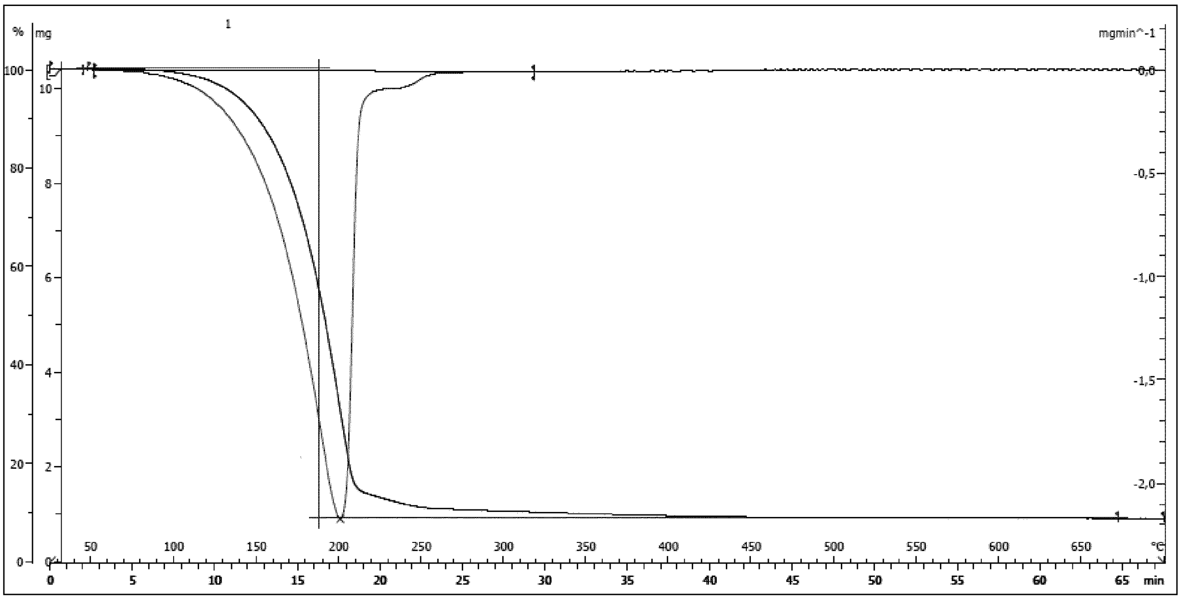
%
mg
1
mgmin^-1
100
80
60
40
20
0
10
8
6
4
2
0
0,0
-0,5
-1,0
-1,5
-2,0
50
100
150
200
250
300
350
400
450
500
550
600
650
°C
0
5
10
15
20
25
30
35
40
45
50
55
60
65
min

FIG. 2

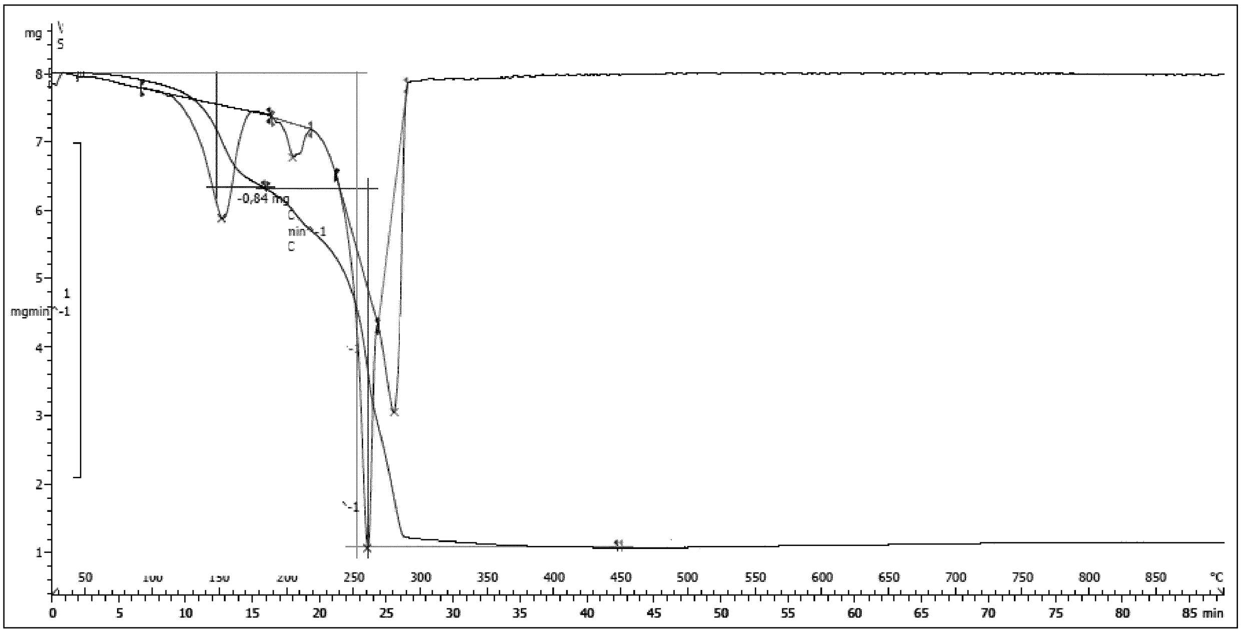
mg
8
7
6
5
4
3
2
1
mgmin^-1
-0,84 mg
50
100
150
200
250
300
350
400
450
500
550
600
650
700
750
800
850
°C
0
5
10
15
20
25
30
35
40
45
50
55
60
65
70
75
80
85 min

FIG. 3

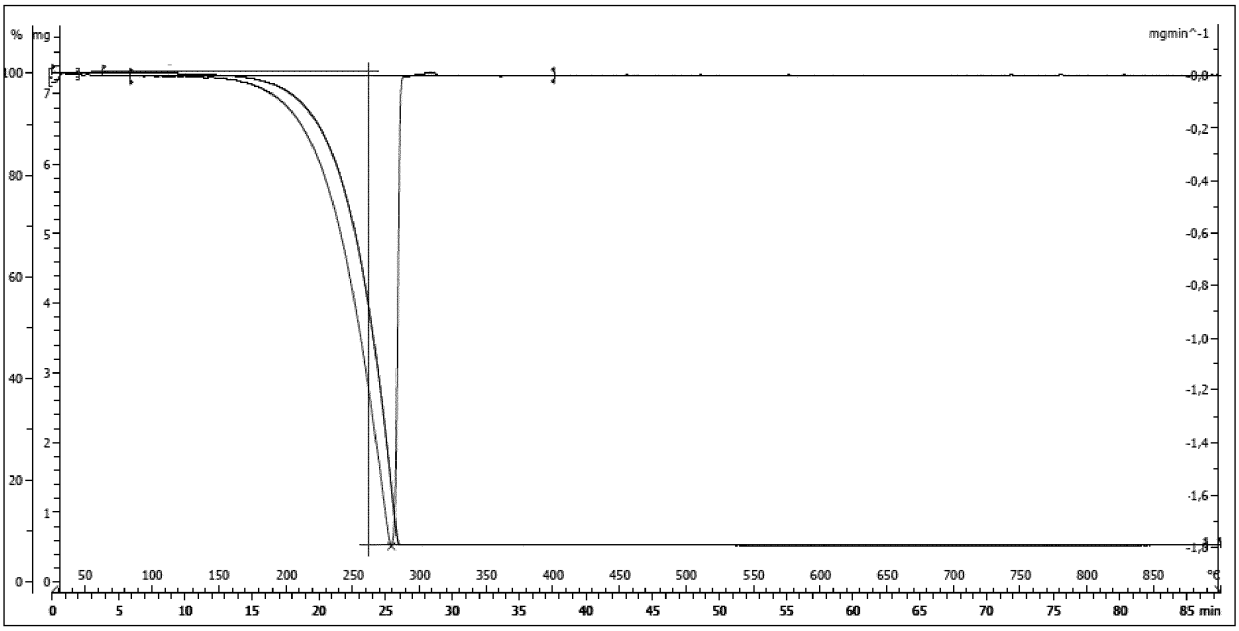
%
mg
mgmin^-1
100
80
60
40
20
0
7
6
5
4
3
2
1
0
-0,0
-0,2
-0,4
-0,6
-0,8
-1,0
-1,2
-1,4
-1,6
-1,8
50 100 150 200 250 300 350 400 450 500 550 600 650 700 750 800 850 °C
0 5 10 15 20 25 30 35 40 45 50 55 60 65 70 75 80 85 min

FIG. 4

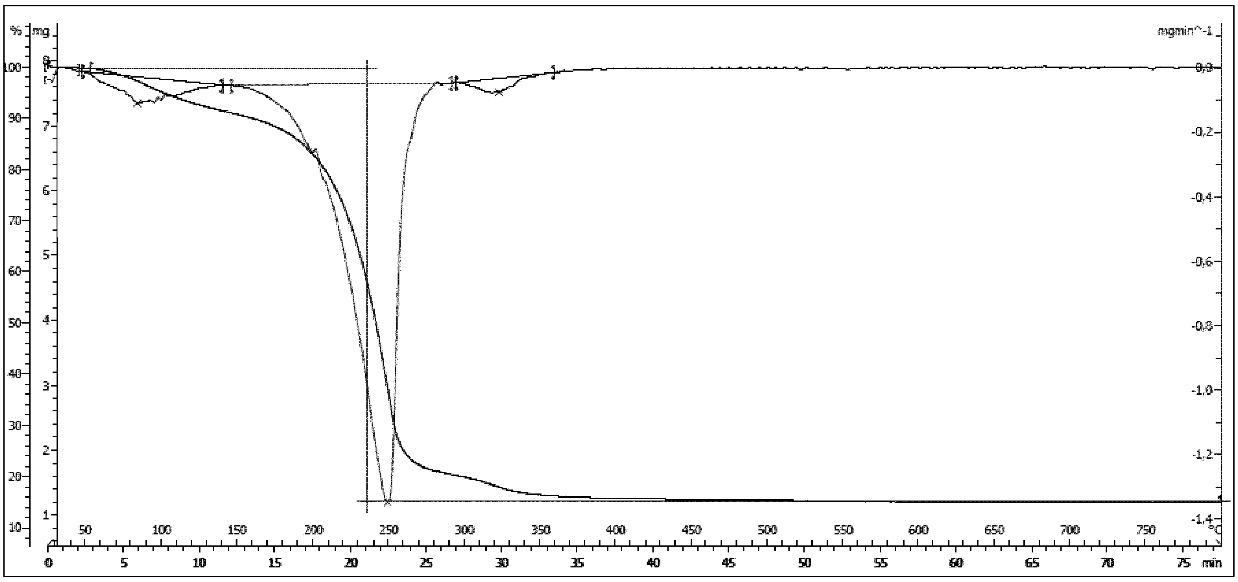

FIG. 5

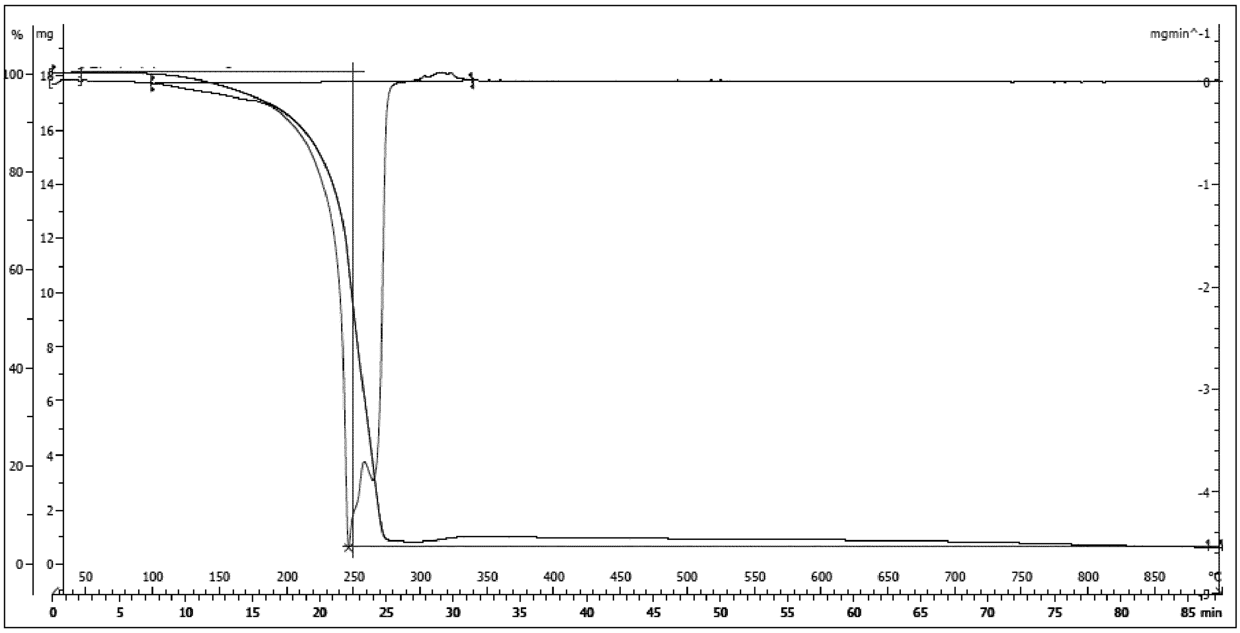
%
mg
mgmin^-1
100
18
16
80
14
12
60
10
8
40
6
4
20
2
0
0
-1
-2
-3
-4
50
100
150
200
250
300
350
400
450
500
550
600
650
700
750
800
850
°C
0
5
10
15
20
25
30
35
40
45
50
55
60
65
70
75
80
85 min

FIG. 6

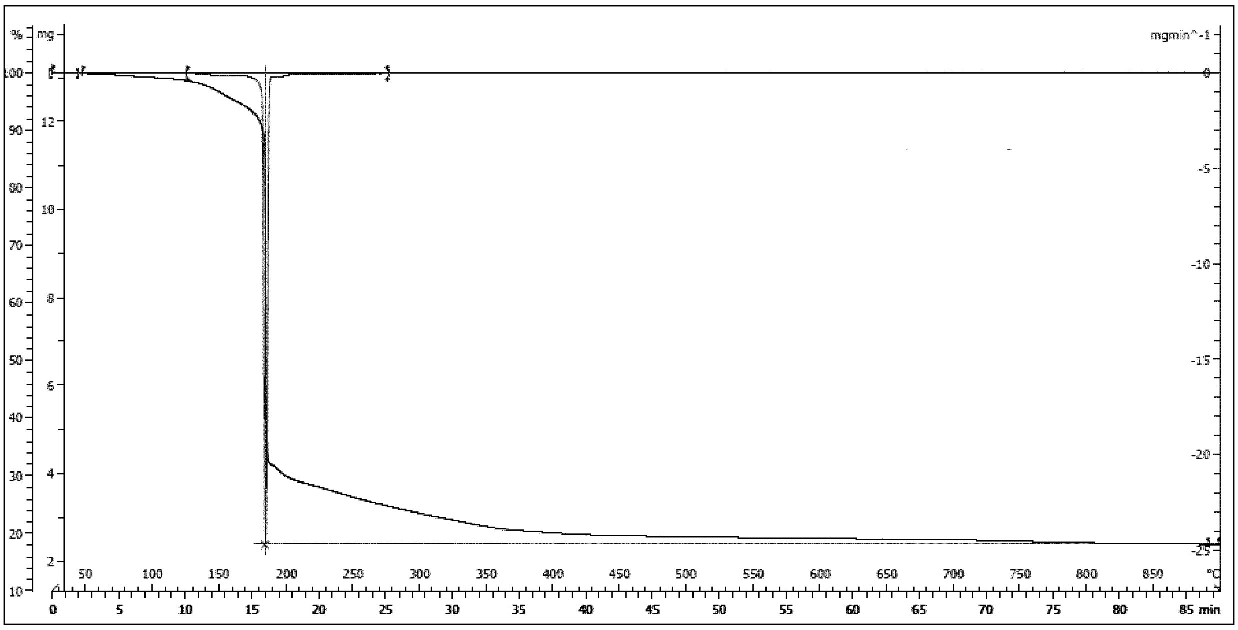
%
mg
mgmin^-1
100
90
80
70
60
50
40
30
20
10
12
10
8
6
4
2
0
-5
-10
-15
-20
-25
50 100 150 200 250 300 350 400 450 500 550 600 650 700 750 800 850 °C
0 5 10 15 20 25 30 35 40 45 50 55 60 65 70 75 80 85 min

FIG. 12

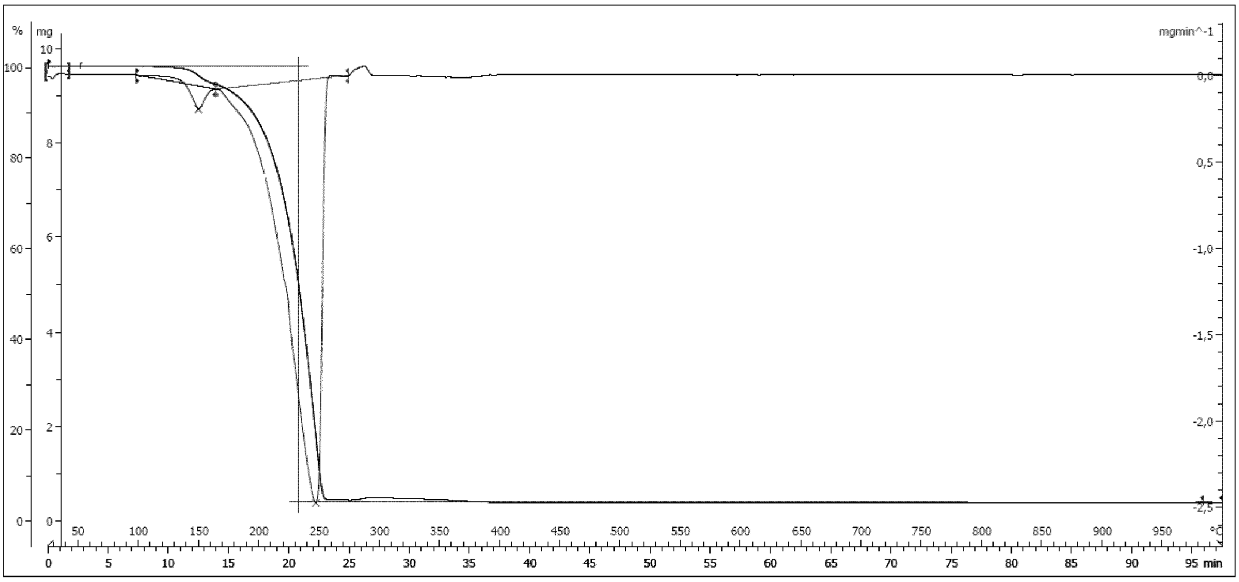
%
mg
mgmin^-1
100
80
60
40
20
0
10
8
6
4
2
0
0,0
-0,5
-1,0
-1,5
-2,0
-2,5
50 100 150 200 250 300 350 400 450 500 550 600 650 700 750 800 850 900 950 °C
0 5 10 15 20 25 30 35 40 45 50 55 60 65 70 75 80 85 90 95 min

FIG. 13

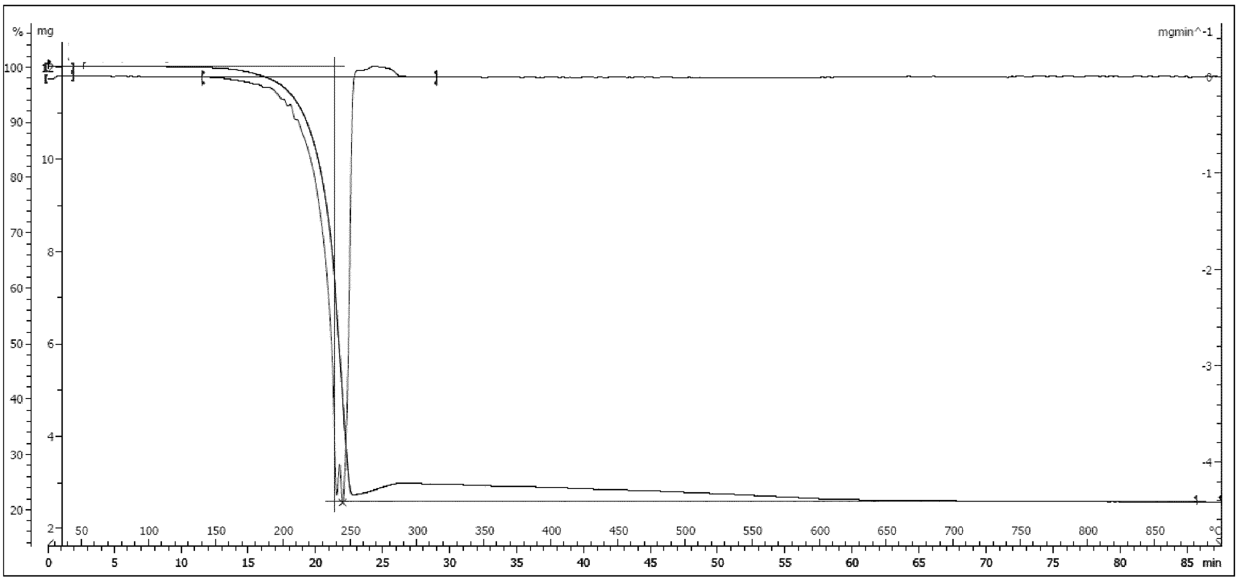

Fig. 21

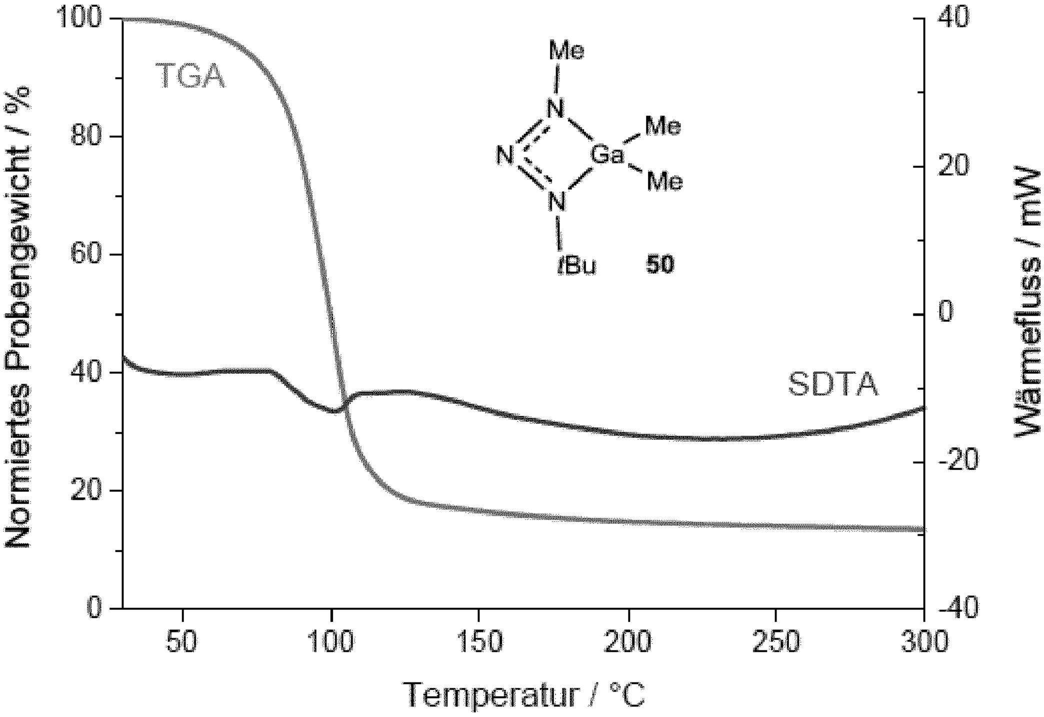
TGA
SDTA
50
Normiertes Probengewicht / %
Wärmefluss / mW
Temperatur / °C
100
80
60
40
20
0
40
20
0
-20
-40
50
100
150
200
250
300

**Fig. 22**

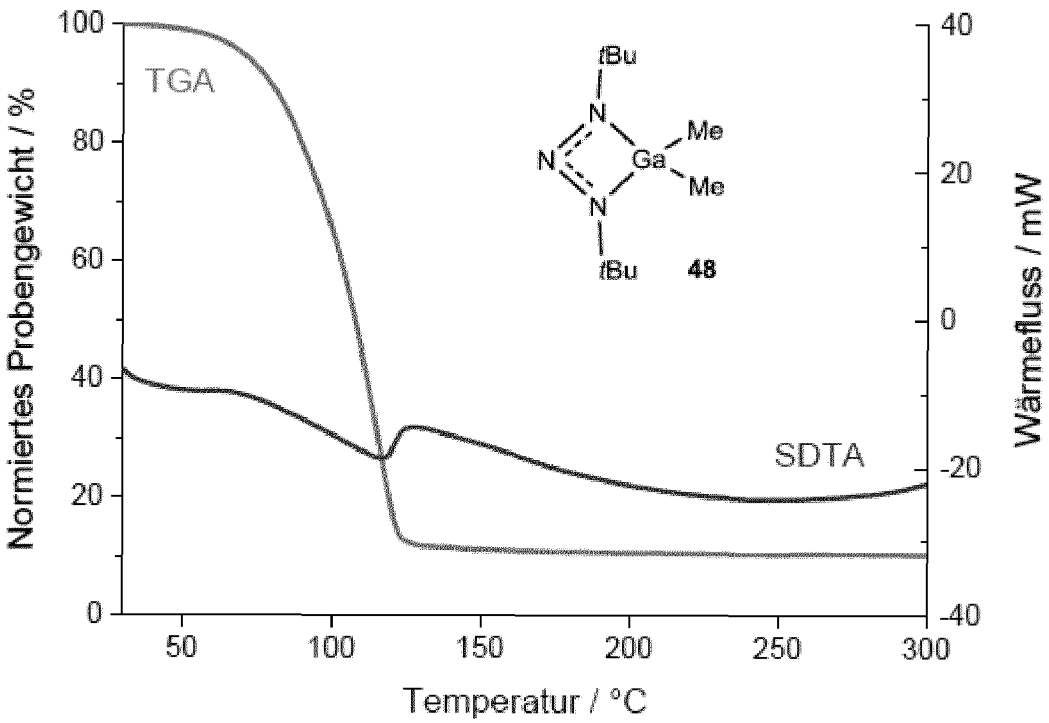
Normiertes Probengewicht / %
100
80
60
40
20
0
TGA
SDTA
48
Wärmefluss / mW
40
20
0
-20
-40
50
100
150
200
250
300
Temperatur / °C

Fig. 23

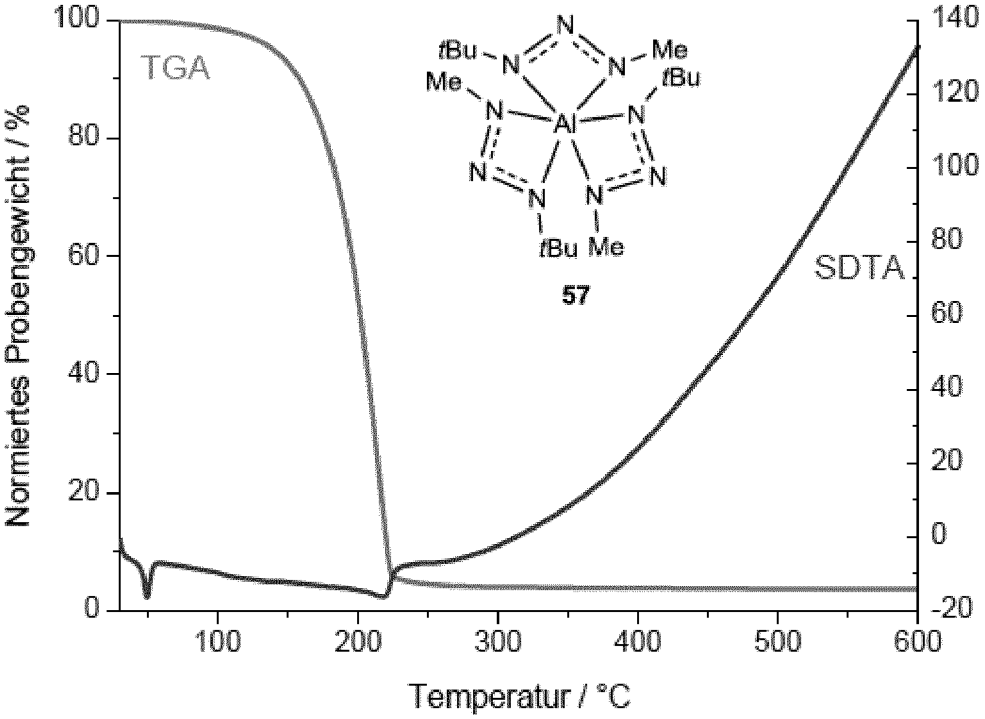
TGA
SDTA
57
Normiertes Probengewicht / %
0
20
40
60
80
100
Wärmefluss / mW
-20
0
20
40
60
80
100
120
140
100
200
300
400
500
600
Temperatur / °C

Fig. 24

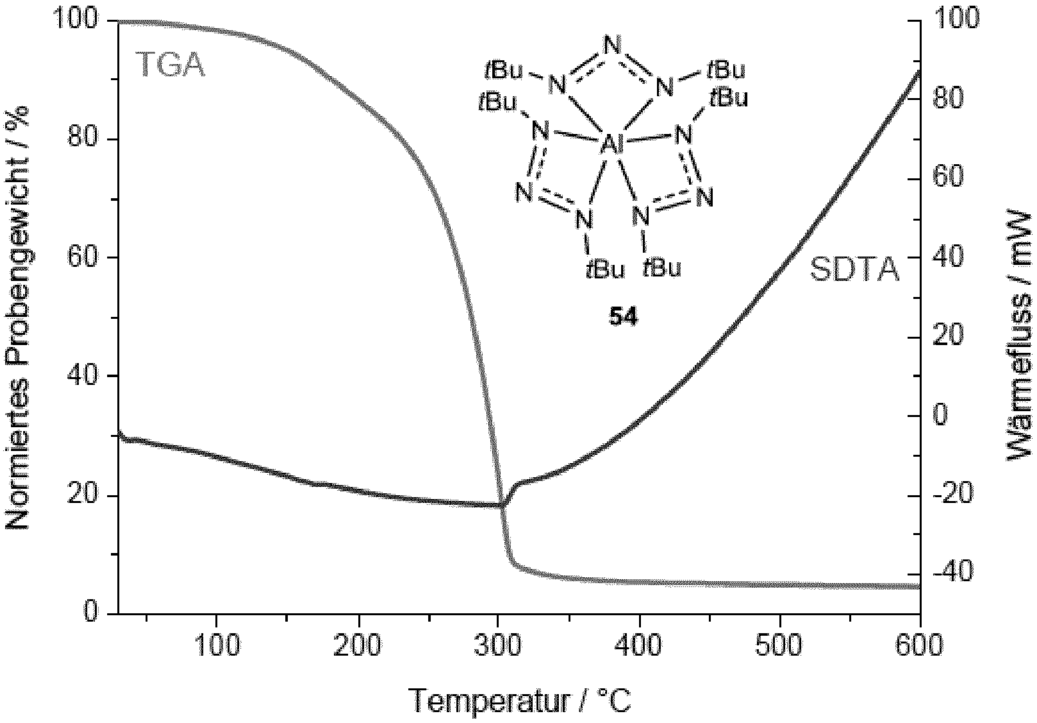
TGA
SDTA
54
Normiertes Probengewicht / %
Wärmefluss / mW
Temperatur / °C
0
20
40
60
80
100
-40
-20
0
20
40
60
80
100
100
200
300
400
500
600

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3386985 A
- WO 2004063233 A, V. C. Gibson, D. F. Reardon, A. K. Tomov


### In der Beschreibung aufgeführte Nicht-Patentliteratur


- **J. ZILKO.** Handbook of Thin Film Deposition - Processes and Technologies. 2001, 151-203
- **OUSSAMA M. El-KADRI et al.** *DALTON TRANSACTIONS,* 01. Januar 2006, 1943-1953
- **DIRK PFEIFFER et al.** *J. of Organomet. Chem.,* 01. Oktober 1999, vol. 588 (2), 167-175
- **E. G. RUKHADZE et al.** *Zhurnal Obshchei Khimii,* 01. Januar 1969, vol. 39 (2), 303-306
- **J. C. BRAND ; B. P. ROBERTS.** *J. Chem. Soc. Chem. Comm.,* 1981, 748-749
- **R. H. J. SMITH ; C. J. MICHEJDA.** *Synthesis,* 1983, 476-477
- **R. H. J. SMITH ; B. D. WLADOWSKI ; A. F. MEHL ; M. J. CLEVELAND ; E. A. RUDROW ; G. N. CHMURNY ; C. J. MICHEJDA.** *J. Org. Chem.,* 1989, vol. 54, 1036-1042
- **D. H. SIEH ; D. J. WILBUR ; C. J. MICHEJDA.** *J. Am. Chem. Soc.,* 1980, 3883-3887
- **SOUSSI, K. ; MISHRA, S ; JEANNEAU, E ; MILLET, J ; M., DANIELE ; S., DALTON.** *Transactions,* 2017, vol. 46 (38), 13055-13064
- **C. LEGO ; B. NEUMÜLLER.** *Z. Anorg. Allg. Chem.,* 2011, vol. 637, 1784-1789
- **P. GANTZEL ; P. J. WALSH.** *Inorg. Chem.,* 1998, vol. 37, 3450-3451
- **D. KALDEN ; S. KRIECK ; H. GÖRLS ; M. WESTERHAUSEN.** *Dalt. Trans.,* 2015, (44), 8089-8099
- **H. S. LEE ; S.-O. HAUBER ; D. VINDUS ; M. NIEMEYER.** *Inorg. Chem.,* 2008, vol. 47, 4401-4412
- **S. L. HYUI ; M. NIEMEYER.** *Inorg. Chem.,* 2006, vol. 45, 6126-6128
- **F. E. BRINCKMAN ; H. S. HAISS ; R. A. ROBB.** *Inorg. Chem.,* 1965, vol. 4, 936-942
- **J. T. LEMAN ; A. R. BARRON ; J. W. ZILLER ; R. M. KREN.** *Polyhedron,* 1989, vol. 8, 1909-1912
- **J. T. LEMAN ; H. A. ROMAN ; A. R. BARRON.** *J. Chem. Soc. Dalt. Trans.,* 1992, vol. 1, 2183
- **M. L. COLE ; A. J. DAVIES ; C. JONES ; P. C. JUNK ; A. I. MCKAY ; A. STASCH.** *Z. Anorg. Allg. Chem.,* 2015, vol. 641, 2233-2244
- **S. G. ALEXANDER ; M. L. COLE ; C. M. FORSYTH ; S. K. FURFARI ; K. KONSTAS.** *Dalt. Trans.,* 2009, 2326-2336
- **J. T. LEMAN ; J. BRADDOCK-WILKING ; A. J. COOLONG ; A. R. BARRON.** *Inorg. Chem.,* 1993, vol. 32, 4324-4336
- **I. A. GUZEI ; L. M. LIABLE-SANDS ; A. L. RHEINGOLD ; C. H. WINTER.** *Polyhedron,* 1997, vol. 16, 4017-4022
- **E. HARTMANN ; J. STRÄHLE.** *J. Inorg. Gen. Chem.,* 1990, vol. 583, 31-40
- **I. D. BROWN ; J. D. DUNITZ.** *Acta Cryst.,* 1961, vol. 14, 480-485
- **L. R. FALVELLO ; E. P. URRIOLABETTIA ; U. MUKHOPADHYAY ; D. RAY.** *Acta Cryst. Comm.,* 1999, vol. C55, 170-172
- **H. S. LEE ; M. NIEMEYER.** *Inorg. Chim. Acta,* 2011, vol. 374, 163-170
- **A. L. JOHNSON ; A. M. WILLCOCKS ; S. P. RICHARDS.** *Inorg. Chem.,* 2009, vol. 48, 8613-8622
- **E. PFEIFFER ; M. W. KOKKES ; K. VRIEZE.** *Transit. Met. Chem.,* 1979, vol. 4, 389-393
- **M. VENTER ; I. HAIDUC ; L. DAVID ; O. COZAR.** *J. Mol. Struct.,* 1997, vol. 408-409, 483-486
- **F. A. COTTON ; X. FENG.** *Inorg. Chem.,* 1989, vol. 28, 1180-1183
- **J. G. M. VAN DER LINDEN ; A. H. DIX, E. PFEIFFER.** *Inorg. Chim. Acta,* 1980, vol. 39, 271-274
- **S. F. COLSON ; S. D. ROBINSON.** *Inorg. Chim. Acta Lett.,* 1988, vol. 149, 13-14
- **S. F. COLSON ; S. D. ROBINSON ; M. MOTEVALLI ; M. B. HURSTHOUSE.** *Polyhedron,* 1988, vol. 7, 1919-1924
- **J. A. CABEZA ; J. M. FERNHDEZ-COLINAS.** *Coord. Chem. Rev.,* 1993, vol. 126, 319-336
- **C. J. WEISS ; T. J. MARKS.** *Dalt. Trans.,* 2010, vol. 39, 6576
- **B. LIU ; D. CUI.** *Dalt. Trans.,* 2009, 550-556
- **P. J. EULGEM ; A. KLEIN ; N. MAGGIAROSA ; D. NAUMANN ; R. W. H. POHL.** *Chem. Eur. J.,* 2008, vol. 14, 3727-3736

- **S. G. ALVAREZ ; M. T. ALVAREZ.** *Synthesis,* 1997, vol. 4, 413-414
- **P. MARGARETHA ; S. SOLAR ; O. E. POLANSKY.** *Angew. Chemie - Int. Ed.,* 1971, vol. 10, 412-413
- **P. MARGARETHA ; S. SOLAR ; O. E. POLANSKY.** *Angew. Chemie,* 1971, vol. 11, 410
- **J. C. BOTTARO ; P. E. PENWELL ; R. J. SCHMITT.** *Synth. Commun.,* 1997, vol. 27, 1465-1467